(19) **Europäisches Patentamt**

**European Patent Office**

**Office européen des brevets**

(11) **EP 0 859 849 B1**

(12) **EUROPEAN PATENT SPECIFICATION**

(45) Date of publication and mention
of the grant of the patent:
**26.07.2006 Bulletin 2006/30**

(51) Int Cl.:
*C12N 15/53* (2006.01)          *C12N 9/06* (2006.01)
*C12N 15/82* (2006.01)          *C12N 5/10* (2006.01)

(21) Application number: **96934039.7**

(22) Date of filing: **03.10.1996**

(86) International application number:
**PCT/US1996/015921**

(87) International publication number:
**WO 1997/012983 (10.04.1997 Gazette 1997/16)**

(54) **POLYPEPTIDES AND POLYNUCLEOTIDES RELATING TO THE ALPHA- and BETA-SUBUNITS OF GLUTAMATE DEHYDROGENASES AND METHODS OF USE**

POLYPEPTIDE UND POLYNUKLEOTIDE BEZÜGLICH ALPHA UND BETA GLUTAMAT-DEHYDROGENASE UNTEREINHEITEN UND DEREN VERWENDUNG

POLYPEPTIDES ET POLYNUCLEOTIDES SE RAPPORTANT AUX SOUS-UNITES ALPHA ET BETA DES GLUTAMATE DESHYDROGENASES ET LEURS METHODES D'UTILISATION

(84) Designated Contracting States:
**AT BE CH DE DK ES FR GB GR IE IT LI NL PT SE**

(30) Priority: **06.10.1995 US 541033**

(43) Date of publication of application:
**26.08.1998 Bulletin 1998/35**

(73) Proprietor: **THE UNIVERSITY OF FLORIDA**
**Gainesville,**
**Florida 32611 (US)**

(72) Inventors:
• **SCHMIDT, Robert, R.**
**Gainesville, FL 32608 (US)**
• **MILLER, Philip**
**Ballwin, MO 63021 (US)**

(74) Representative: **Perry, Robert Edward et al**
**Gill Jennings & Every LLP**
**Broadgate House**
**7 Eldon Street**
**London EC2M 7LH (GB)**

(56) References cited:
**WO-A-95/09911          WO-A1-95/09911**

• **PLANT MOL. BIOL. (1991), 17(5), 1023-44 CODEN: PMBIDB;ISSN: 0167-4412, XP002024794 COCK, J. MARK ET AL: "A nuclear gene with many introns encoding ammonium-inducible chloroplastic NADP-specific glutamate dehydrogenase (s) in Chlorella sorokiniana"**

• **CHEMICAL ABSTRACTS, vol. 106, no. 21, 25 May 1987 Columbus, Ohio, US; abstract no. 171680, BASCOMB, NEWELL F. ET AL: "Purification and partial kinetic and physical characterization of two chloroplast-localized NADP-specific glutamate dehydrogenase isoenzymes and their preferential accumulation in Chlorella sorokiniana cells cultured at low or high ammonium levels" XP002024795 & PLANT PHYSIOL. (1987), 83(1), 75-84 CODEN: PLPHAY; ISSN: 0032-0889,**

• **DATABASE WPI Section Ch, Week 8330 Derwent Publications Ltd., London, GB; Class B02, AN 83-721194 XP002024797 & SU 958 502 A (BIOCHEM INST), 15 September 1982**

• **PELZER-REITH B., ET AL.: "Plant aldolase: cDNA and deduced amino-acid sequences of the chloroplast and cytosol enzyme from spinach" PLANT MOLECULAR BIOLOGY, KLUWER ACADEMIC, vol. 21, 1993, pages 331-340, XP009004627**

• **PRUNKARD D. ET AL.: "Evidence for chloroplastic localization of an ammonium-inducible glutamate dehydrogenase and synthesis of its subunit from a cytosolic prcursor protein in Chlorella sorokiana" PLANT PHYSIOLOGY, vol. 81, no. 2, 1986, pages 349-355, ROCKVILLE, US**

EP 0 859 849 B1

**Description**

Background of the Invention

Inorganic nitrogen acquired by plants is ultimately converted to ammonia before being assimilated in organic nitrogen metabolism. One enzyme postulated to be involved in the assimilatory process is glutamate dehydrogenase (GDH), a group of ubiquitous enzymes found to be present in almost all organisms from microbes to higher plants and animals (Srivastava, H.S., R.P. Singh [1987] *Phytochem.* 26:597-610). GDH catalyses the reversible conversion of α-ketoglutarate to glutamate via a reductive amination that utilizes reduced β-nicotinamide adenine dinucleotide (NADH) or reduced β-nicotinamide adenine dinucleotide phosphate (NADPH) as a cofactor. The role of plant GDHs in the assimilation of ammonia into amino acids has been questioned since the discovery of the glutamine synthetase/glutamate synthase (GS/GOGAT) pathway that is believed to be the favored pathway for ammonia assimilation in higher plants (Miflin, BJ., P.J. Lea [1976] *Phytochem.* 15:873-885).

The primary objection to GDH playing a major role in plant nitrogen metabolism is its low affinity for ammonia that would require high intracellular ammonia concentrations to function anabolically. Early evidence indicated that GDH is a catabolic enzyme catalyzing the deamination of glutamate with only a partially anabolic function in synthesizing glutamate (Wallgrove, J.C., N.P. Hall, A.C. Kendall, [1987] *Plant Physiol.* 83:155-158). The physiological role of large amounts of GDH present in various plant tissues and organelles is still unclear, and possible conditions under which GDH may play a significant role in carbon and nitrogen metabolism have not been resolved.

The majority of plant GDHs characterized to date are localized in the mitochondria; however, a GDH species differing in several properties (e.g., cofactor specificity, $K_m$ values, organelle localization, thermal stability, among others) has been characterized from the chloroplast of a unicellular green alga *Chlorella sorokiniana. C. sorokiniana* cells have been shown to possess a constitutive, mitochondrial, tetrameric NAD-specific GDH (hereinafter designated "NAD-GDH") (Meredith, MJ., RM. Gronostajski, R.R. Schmidt [1978] *Plant Physiol.* 61:967-974), and seven ammonium-inducible, chloroplast-localized, homo- and heterohexameric NADP-specific GDH isoenzymes (hereinafter designated "NADP-GDH")(Prunkard, D.E., N.F. Bascomb, R.W. Robinson, R.R. Schmidt [1986] *Plant Physiol.* 81:349-355; Bascomb, N.F., R.R. Schmidt [1987] *Plant Physiol.* 83:75-84). The seven chloroplastic NADP-GDH isoenzymes were shown to have different electrophoretic mobilities during native-PAGE, which can result from the formation of homo- and heterohexamers composed of varying ratios of α- and β-subunits (53.5 and 52.3 kilodaltons, respectively).

*Chlorella* cells cultured in 1 to 2 mM ammonium medium accumulate only the α-homohexamer (Bascomb and Schmidt, *supra*). The addition of higher ammonium concentrations (3.4 to 29 mM) to nitrate-cultured cells results in the accumulation of both α- and β-subunits in NADP-GDH holoenzymes (Prunkard *et al., supra;* Bascomb and Schmidt, *supra;* Bascomb, N.F., D.E. Prunkard, R.R. Schmidt [1987] *Plant Physiol.* 83:85-91). Prunkard *et al.* (Prunkard, D.E., N.F. Bascomb, NF, W.T. Molin, R.R. Schmidt [1986] *Plant Physiol.* 81:413-422) demonstrated that the NADP-GDH subunit ratio and isoenzyme pattern is influenced by both the carbon and nitrogen source as well as the light conditions under which cells are cultured.

The α- and β-NADP-GDH homohexamers purified from *Chlorella* cells have strikingly different ammonium $K_m$ values; however, the $K_m$ values for their other substrates are very similar. The α-homohexamer (composed of six identical α-subunits) that catalyzes the biosynthesis of glutamate is allosterically regulated by NADPH and possesses an unusually low $K_m$ for ammonium that ranges from 0.02 to 3.5 mM, depending on the NADPH concentration (Bascomb and Schmidt, *supra*). The $K_m$ value for ammonium of the α-homohexamer is the lowest reported ammonium $K_m$ for any plant GDH characterized to date. In contrast, the β-homohexamer (catabolic form) is a non-allosteric enzyme with an ammonium $K_m$ of approximately 75 mM. From these studies involving purified enzymes, it had been heretofore postulated that the heterohexamers have varying degrees of affinity for ammonium ranging between the $K_m$ values for the α- and β-homohexamers. Surprisingly, however, we have discovered that certain heterohexamers can have aminating:deaminating activity ratio which is greater than either the α- or β-homohexamers.

Although the α- and β-subunits have distinct *in vivo* turnover rates (Bascomb *et al., supra*) and the corresponding homohexamers have remarkably different ammonium $K_m$ values, the α- and β-subunits are derived from precursor proteins of nearly identical size (ca 58,000 Daltons) and were shown to have very similar peptide maps (Prunkard *et al., supra;* Bascomb and Schmidt, *supra*). Moreover, polyclonal antibodies prepared against the β-homohexamer are capable of immunoprecipitating of all the NADP-GDH isoenzymes (Yeung *et al* [1981] *Anal. Biochem.* 10:216-228; Bascomb *et al., supra*), but do not crossreact with the mitochondrial NAD-GDH.

Cock et al [1991] *Plant Mol. Biol.* 17:17-27, describes a nucleotide sequence encoding the entire mature β-subunit of glutamate-dehydrogenase (GDH) of *Chlorella sorokiniana* and at least 98% of the mature α-subunit . This article also provides genomic cloning and Southern blot evidence indicating that the *C. sorokiniana* genome possesses a single NADP-GDH structural gene.

The *C. sorokiniana* nuclear-encoded chloroplastic NADP-GDH isoenzymes are the only chloroplastic localized GDH sequences isolated and characterized from plants.

## Summary of the Invention

It has now been discovered that the two mature subunits of *Chlorella* GDH isoenzymes arise via specific processing of two similar precursor proteins encoded by two mRNAs formed by alternative splicing of a pre-mRNA derived from a single nuclear gene. Furthermore, the cleavage site and amino-terminal peptide sequence of the mature functional GDH subunits have been identified.

More specifically, the present invention provides the isolation and characterization of two full-length cDNAs from mRNAs isolated from the unicellular green algae *Chlorella sorokiniana.* The two cDNAs encode the precursor proteins (α-precursor, 56.35 kD; β-precursor, 57.85 kD) that are processed to yield the mature α- and β-subunits (53.5 kD; 52.3 kD, respectively) that compose the active NADP-GDH hexameric isoenzymes. The present invention concerns a single NADP-GDH gene which is alternatively spliced to yield two mRNAs that encode two different chloroplast precursor proteins. These precursor proteins can then be processed to the mature α- and β-subunits of the NADP-GDH isoenzymes. Also described are useful fragments or mutants of the nucleotide and amino acid sequences which retain the disclosed activity or utility. For example, certain fragments of the amino acid sequences provided herein can be useful as transit peptides, providing the protein with the capability to enter and remain in certain cell compartments. The nucleotide sequences which are described herein, and fragments of those nucleotide sequences, can be useful, for example, as primers in amplification procedures or as probes to hybridize to complementary sequences of interest. The nucleotide and amino acid sequences and fragments thereof as described herein can also be useful as molecular weight markers or in identifying and conforming the relatedness of other nucleotide sequences, polypeptides, or isoenzymes which pertain to NADP-GDH.

The present invention further provides methods in which assimilation of inorganic nitrogen into organic nitrogen metabolism of higher plants can be altered by expressing GDH from *C. sorokiniana* or GDHs isolated from other organisms. The alteration of nitrogen assimilation can have the effect of increasing nitrogen assimilation which, as is well understood in the an, can affect the composition of the plant through an inverse effect on carbon metabolism, e.g., accumulation of carbohydrates. The subject invention also concerns DNA constructs for use in the described methods. The present invention includes the identification of the amino-terminal sequences of the α- and β-subunits which can assemble to form NADP-GDH isoenzymes, e.g., the native hexameric NADP-GDH found in *C. sorokiniana* chloroplasts. This precise molecular information can be employed to express NADP-GDH with the unique kinetic properties of the *C. sorokiniana* chloroplastic α- and β-NADP-ODH homohexamers. The present invention also provides recombinant cells or organisms, e.g., transgenic crops or plants which, by expressing the genes of the described polynucleotide sequences to produce corresponding polypeptides, can have an increased yield, improved ammonia assimilatory properties which can advantageously increase their tolerance of ammonia toxicity, improved osmotic stress tolerance, and improved composition of the crop or plant.

## Brief Description of the Drawings

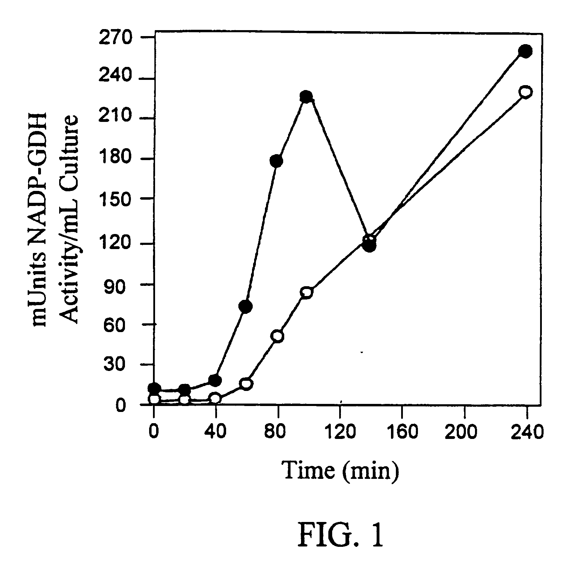

**Figure 1** shows a pattern of NADP-GDH activities in homogenates of synchronous *C. sorokiniana* cells cultured for 240 min in 29 mM ammonium medium in continuous light. Aliquots of clarified homogenates, from cell collected at various time intervals, were analyzed spectrophotometrically for both aminating (●) and deaminating (o) NADP-GDH activities.

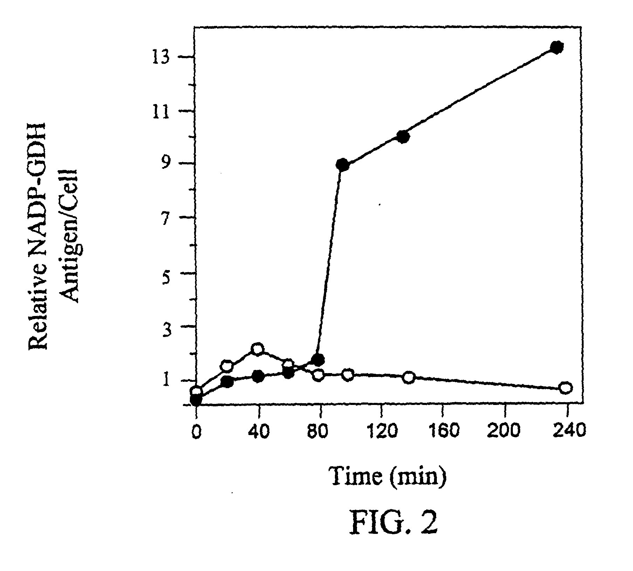

**Figure 2** shows patterns of accumulation of NADP-GDH antigens in illuminated cells cultured in 29 mM ammonium medium for 240 min. At zero time, ammonium was added to synchronous *C. sorokiniana* daughter cells and the culture was illuminated. Autoradiographs of Western blots were analyzed by laser densitometry to determine the relative levels of the NADP-GDH α-subunit (●) and β-subunit (o) throughout the 240 min induction period.

## Brief Description of the Sequences

**SEQ ID NO. 1** is the cDNA for the precursor-protein of the α-subunit of an NADP-specific glutamate dehydrogenase.
**SEQ ID NO. 2** is the deduced amino acid sequence of the polynucleotide of SEQ ID NO. 1.
**SEQ ID NO. 3** is the cDNA for the precursor-protein of the β-subunit of an NADP-specific glutamate dehydrogenase.
**SEQ ID NO. 4** is the deduced amino acid sequence of the polynucleotide of SEQ ID NO. 3.
**SEQ ID NO. 5** is the N-terminal sequence for the NADP-GDH α-subunit.
**SEQ ID NO. 6** is the N-terminal sequence for the NADP-GDH β-subunit.
**SEQ ID NO. 7** is the cDNA sequence in the clone designated pBGDc53.

**SEQ ID NO. 8 is** a primer which hybridizes to the conserved region of NADP-GDH mRNAs.

**SEQ ID NO. 9** is a poly(dT) polynucleotide used as an adaptor primer according to the subject invention.

**SEQ ED NO. 10** is a polynucleotide used as a primer according to the subject invention.

**SEQ ID NO. 11** is a polynucleotide used as a primer according to the subject invention.

**SEQ ID NO. 12** is a polynucleotide used as an adaptor primer according to the subject invention.

**SEQ ID NO. 13** is the polynucleotide insert in the clone designated pRGDc 60.

**SEQ ID NO. 14** is the polynucleotide insert in the clone designated pRGDc 61.

**SEQ ID NO. 15** is the polynucleotide used as a primer according to the subject invention.

**SEQ ID NO. 16** is the polynucleotide insert in a clone designated pGDc 63.

**SEQ ID NO. 17** is the polynucleotide insert of a clone designated pGDc 64.

**SEQ ID NO. 18** is the polynucleotide resulting from ligation of purified fragments of the inserts in the clones designated pBGDc 53 and pGDc 63, according to the subject invention.

**SEQ ID NO. 19** is the polynucleotide resulting from ligation of purified inserts of the clones designated pGDc 64 and pBGDc 53.

**SEQ ID NO. 20** is a polynucleotide used as a primer according to the subject invention.

**SEQ ID NO. 21** is a polynucleotide used as a primer hybridizing to the 3' terminus of the template DNA according to the subject invention.

**SEQ ID NO. 22** is a polynucleotide used as a primer according to the subject invention.

**SEQ ID NO. 23** is the polynucleotide sequence (cDNA) of the processed, mature NADP-GDH α-subunit.

**SEQ ID NO. 24** is the amino acid sequence of the processed, mature NADP-GDH α-subunit.

**SEQ ID NO. 25** is the polynucleotide (cDNA) sequence of the processed, mature NADP-GDH β-subunit.

**SEQ ID NO. 26 is** the amino acid sequence of the processed, mature NADP-GDH β-subunit.

Detailed Disclosure of the Invention

The present invention provides heretofore undescribed polynucleotide sequences, for example, cDNAs for precursor-proteins of α- and β-subunits of an ammonium inducible, chloroplast-localized NADP-specific glutamate dehydrogenase (hereinafter NADP-GDH) from *Chlorella sorokiniana.* The nucleotide sequences for the precursor proteins of the α- and β-subunits that form NADP-GDH are shown in SEQ ID NOS. 1 and 3, respectively. The deduced amino acid sequences for the precursor-proteins of the α- and β-subunits of the NADP-GDH enzyme from *Chlorella sorokiniana* are shown in SEQ ID NOS. 2 and 4, respectively.

*E. coli* hosts comprising the subject cDNA inserts were deposited with the American Type Culture Collection (ATCC), 12301 Parklawn Drive, Rockville, Maryland 20852 USA. The cultures were assigned the following accession numbers by the repository:

| Culture | Accession number | Deposit date |
|---|---|---|
| *E. coli* DH5α α-NADP-GDH SEQ No. 1 (+42 bp) | ATCC 69925 | October 6, 1995 |
| *E. coli* DH5α β-NADP-GDH SEQ No. 1 (-42 bp) | ATCC 69926 | October 6, 1995 |

Automated amino acid sequence analysis identifies 20 and 10 amino-terminal amino acid residues of the α- and β-subunits, respectively. Alignment of the α- and β-subunit peptide sequences reveals that the two subunits are identical with the exception of an 11-amino acid extension present in the larger α-subunit. Monoclonal antibodies raised against the α-subunit were shown to recognize the β-subunit providing further evidence that the two subunits are nearly identical. The identification of the unique α- and β-subunit processing sites within the precursor proteins provides the molecular mechanism to explain the different kinetic properties of the α- and β-NADP-GDH homohexameric isoenzymes.

The aforementioned data provide information applicable to genetically engineer plants with a specific GDH having favorable kinetic properties which can influence both carbon and nitrogen metabolism. Based on the high guanine/cytosine content the cDNAs are highly amenable for heterologous expression in higher plants. The introduction of either or both subunits with their chloroplast targeting sequences or with other organellar targeting sequences in heterologous plant systems can improve nitrogen assimilation and influence the carbon/nitrogen balance.

It has been discovered that chloroplast localization is related to, and can be dependent on, the N-terminus of the α- or β-precursor protein. Cleavage of the N-terminus of the precursors yields the mature proteins. Accordingly, the chloroplast transit peptide comprises a peptide which forms, or is an active fragment of, the N-terminus cleaved from the precursor protein. Peptides having similar or equivalent amino acid sequences, or that have a tertiary structure or conformation similar to these cleaved peptides can also function as transit peptides. The chloroplast-transit peptide comprises the active fragment of the N-terminal peptide cleaved from the α-precursor (a 40-mer) or the β-precursor (a 37-mer). The polynucleotide sequences encoding the chloroplast-transit peptides can be used by persons of ordinary skill in the art to produce chloroplast-transit peptides employed with the peptides described herein, or others known in the art.

Adding, removing, or replacing the chloroplast-transit peptide associated with a protein, e.g., the GDH enzyme, can be employed to localize the protein according to need, by means well known in the art. For example, localization of the enzyme in a chloroplast of a cell can be achieved by the insertion of a chloroplast-transit peptide onto an amino acid sequence lacking such a transit peptide. Species-specific chloroplast-transit peptides can be added or can replace those present to optimize insertion into the chloroplast of a desired species. In addition, localization inside the chloroplast of a protein expressed within the chloroplast can be achieved by direct transformation of the plastid with the polynucleotide sequences encoding an expressed protein. Similarly, removal of a chloroplast-transit peptide or production of a recombinant protein lacking the peptide can be utilized to sequester the protein in a cellular compartment other than the chloroplast.

Transformed plants expressing the $\alpha$-homohexamer can be more tolerant to ammonia toxicity, assimilate ammonium more efficiently, and respond more rapidly to osmotic stress encountered in transiently saline soils by providing glutamate the precursor to the osmoprotectant proline. Expression of, for example, the $\beta$-homohexamer or GDH heterohexamers can be used to alter the rate of nitrogen assimilation, favoring accumulation of carbohydrates in fruits and other storage organs.

Unexpectedly, it was discovered that a hexamer comprising at least one $\alpha$-subunit and at least one $\beta$-subunit, i.e., a heterohexamer, can have advantageous activity. Specifically, the aminating:deaminating activity ratio (i.e., biosynthetic capacity for synthesis of glutamate) of a chloroplastic NADP-GDH isozyme can be increased by incorporating both $\alpha$- and $\beta$-subunits into the hexameric protein rather than using a homohexamer comprising only the $\alpha$- or only the $\beta$-subunits. In one embodiment of the invention, it can be advantageous to co-express cDNAs encoding both types of subunits in the same plant at different rates/levels such that a particular ratio of $\alpha$- and $\beta$-subunits is obtained in the heterohexamer. For example, we have discovered that an NADP-GDH heterohexamer having at least one of the subunits in the $\beta$-form is preferred for increasing aminating:deaminating activity ratio. A more preferred heterohexamer has 2-5 $\beta$-subunits. This differential rate of expression of the two cDNAs can be accomplished by placing them under the control of plant promoters with different strengths or under the same promoter that has been modified to generate different levels of expression. The use of this algal NADP-GDH isozyme system in plant biotechnology has advantages over NADP-GDHs from organisms, such as bacteria, that contain only a single form of the enzyme (i.e., no isozymes).

It is recognized that expression levels of certain recombinant proteins in transgenic plants can be improved via increased expression of stabilized mRNA transcripts; and that, conversely, detection of these stabilized RNA transcripts may be utilized to measure expression of translational product (protein). Low expression of protein RNA in plants and, therefore, of low protein expression, can be resolved through the use of an improved, synthetic gene specifying the desired protein from the gene source organism.

Thus, in one embodiment of the subject invention, bacteria and plants can be genetically engineered to attain desired expression levels of novel proteins having agricultural or otherwise commercial value. To provide genes having enhanced expression in plants, the DNA sequence of the gene can be modified to comprise codons preferred by highly expressed plant genes, to attain an A+T content in nucleotide base composition substantially that found in plants, and also preferably to form a plant initiation sequence, and to eliminate sequences that cause destabilization, inappropriate polyadenylation, degradation and termination of RNA and to avoid sequences that constitute secondary structure hairpins and RNA splice sites. For example, in synthetic genes, the codons used to specify a given amino acid can be selected with regard to the distribution frequency of codon usage employed in highly expressed plant genes to specify that amino acid. As is appreciated by those skilled in the art, the distribution frequency of codon usage utilized in the synthetic gene is a determinant of the level of expression.

For purposes of the subject invention, "frequency of preferred codon usage" refers to the preference exhibited by a specific host cell in usage of nucleotide codons to specify a given amino acid. To determine the frequency of usage of a particular codon in a gene, the number of occurrences of that codon in the gene is divided by the total number of occurrences of all codons specifying the same amino acid in the gene. Similarly, the frequency of preferred codon usage exhibited by a host cell can be calculated by averaging frequency of preferred codon usage in a large number of genes expressed by the host cell. It is preferable that this analysis be limited to genes that are highly expressed by the host cell.

When synthesizing a gene for improved expression in a host cell it is desirable to design the gene such that its frequency of codon usage approaches the frequency of preferred codon usage of the host cell.

The percent deviation of the frequency of preferred codon usage for a synthetic gene from that employed by a host cell is calculated first by determining the percent deviation of the frequency of usage of a single codon from that of the host cell followed by obtaining the average deviation over all codons. As defined herein this calculation includes unique codons (i.e., ATG and TGG). In general terms the overall average deviation of the codon usage of a synthetic gene from that of a host cell is calculated using the equation

$$A = \sum_{n=1}^{Z} \frac{\frac{X_n - Y_n}{X_n} \, x \, 100}{Z}$$

where $X_n$=frequency of usage for codon n in the host cell; $Y_n$=frequency of usage for codon n in the synthetic gene. Where n represents an individual codon that specifies an amino acid, the total number of codons is Z. The overall deviation of the frequency of codon usage, A, for all amino acids should preferably be less than about 25%, and more preferably less than about 10%. Hence, a gene can be designed such that its distribution frequency of codon usage deviates, preferably, no more than 25% from that of highly expressed plant genes and, more preferably, no more than about 10%. In addition, consideration is given to the percentage G+C content of the degenerate third base (monocotyledons appear to favor G+C in this position, whereas dicotyledons do not). It is also recognized that the XCG (where X is A, T, C or G) nucleotide is the least preferred codon in dicots whereas the XTA codon is avoided in both monocots and dicots. Synthetic genes of this invention also preferably have CG and TA doublet avoidance indices closely approximating those of the chosen host plant. More preferably these indices deviate from that of the host by no more than about 10-15%.

Assembly of the NADP-GDH gene of this invention can be performed using standard technology known in the art. A structural gene designed for enhanced expression in plants of the specific embodiment can be enzymatically assembled within a DNA vector from chemically synthesized oligonucleotide duplex segments. The gene can then be introduced into a plant host cell and expressed by means known to the art. Preferably, the protein produced upon expression of the synthetic gene in plants is functionally equivalent to a native protein in having comparable or improved aminating/deaminating activity. According to the subject invention, *functionally equivalent* refers to identity or near identity of function. A synthetic gene product which has at least one property relating to its activity or function, which is the same or similar to a natural protein is considered functionally equivalent thereto. Modifications in nucleotide sequence of the coding region can be made to alter the A+T content in DNA base composition of a synthetic gene to reflect that normally found in genes for highly expressed proteins native to the host cell. Preferably the A+T content of the synthetic gene is substantially equal to that of said genes for highly expressed proteins. In genes encoding highly expressed plant proteins, the A+T content is approximately 55%. It is preferred that the synthetic gene have an A+T content near this value, and not sufficiently high as to cause destabilization of RNA and, therefore, lower the protein expression levels. More preferably, the A+T content is no more than about 60% and most preferably is about 55%. Also, for ultimate expression in plants, the synthetic gene nucleotide sequence preferably can be modified to form a plant initiation sequence at the 5' end of the coding region. In addition, particular attention is preferably given to assure that unique restriction sites are placed in strategic positions to allow efficient assembly of oligonucleotide segments during construction of the synthetic gene and to facilitate subsequent nucleotide modification. As a result of these modifications in coding region of the native gene, the preferred synthetic gene is expressed in plants at an enhanced level when compared to that observed with natural structural genes.

It is known that the relative use of synonymous codons differs between the monocots and the dicots. In general, the most important factor in discriminating between monocot and dicot patterns of codon usage is the percentage G+C content of the degenerate third base. In monocots, 16 of 18 amino acids favor G+C in this position, while dicots only favor G+C in 7 of 18 amino acids.

For soybean and maize, the maize codon usage pattern resembles that of monocots in general, whereas the soybean codon usage pattern is almost identical to the general dicot pattern.

In designing a synthetic gene for expression in plants, it is preferred to eliminate sequences which interfere with the efficacy of gene expression.

A synthetic gene may be synthesized for other purposes in addition to that of achieving enhanced levels of expression. For example, in accordance with the subject invention, one of the nucleotide sequences encoding the α-subunit or the β-subunit of NADP-GDH can be modified such that the products are differentially expressed, favoring expression of one of the subunits. A result of such differential expression is a heterohexamer comprising more of one subunit than the other. Modification may encompass substitution of one or more, but not all, of the oligonucleotide segments used to construct the synthetic gene by a corresponding region of natural sequence. Preferably, differential expression of the nucleotide sequences encoding the α- and β-subunits of the NADP-GDH polypeptides can be employed to produce a heterohexamer having at least one β-subunit, more preferably two to five β-subunits, and most preferably three β-subunits.

The recombinant DNA molecule comprising a nucleotide sequence of the subject invention can be introduced into plant tissue by any means known to those skilled in the art. The technique used for a given plant species or specific type of plant tissue depends on the known successful techniques. As novel means are developed for the stable insertion of foreign genes into plant cells and for manipulating the modified cells, skilled artisans will be able to select from known means to achieve a desired result. Means for introducing recombinant DNA into plant tissue include, but are not limited to, direct DNA uptake (Paszkowski, J. et al. (1984) EMBO J. 3:2717), electroporation (Fromm, M. et al. (1985) Proc. Natl. Acad. Sci. USA 82:5824), microinjection (Crossway, A. et al. (1986) Mol. Gen. Genet. 202:179), or T-DNA mediated transfer from *Agrobacterium tumefaciens* to the plant tissue. There appears to be no fundamental limitation of T-DNA transformation to the natural host range of Agrobacterium. Successful T-DNA-mediated transformation of monocots (Hooykaas-Van Slogteren, G. et al. (1984) Nature 311:763), gymnosperms (Dandekar, A. et al. (1987) Biotechnology 5:587) and algae (Ausich, R., EPO application 108,580) has been reported. Representative T-DNA vector systems are described in the following references: An, G. et al. (1985) EMBO J. 4:277; Herrera-Estrella, L. et al. (1983) Nature 303: 209; Herrera-Estrella, L. et al. (1983) EMBO J. 2:987; Herrera-Estrella, L. et al. (1985) in *Plant Genetic Engineering,* New York: Cambridge University Press, p. 63. Once introduced into the plant tissue, the expression of the structural gene may be assayed by any means known to the art, and expression may be measured as mRNA transcribed or as protein synthesized. Techniques are known for the in vitro culture of plant tissue, and in a number of cases, for regeneration in to whole plants. Procedures for transferring the introduced expression complex to commercially useful cultivars are known to those skilled in the art.

In one of its preferred embodiments the invention disclosed herein comprises expression in plant cells of an NADP-GDH gene under control of a plant expressible promoter, that is to say, by inserting the gene into T-DNA under control of a plant expressible promoter and introducing the T-DNA containing the insert into a plant cell using known means. Once plant cells expressing the gene under control of a plant expressible promoter are obtained, plant tissues and whole plants can be regenerated therefrom using methods and techniques well-known in the art. The regenerated plants are then reproduced by conventional means and the introduced genes can be transferred to other strains and cultivars by conventional plant breeding techniques.

The introduction and expression of the NADP-GDH gene can be used to improve, e.g., increase, yields in a crop. Other uses of the invention, exploiting the properties of the genes introduced into plant species will be readily apparent to those skilled in the art.

Differences also exist between codon choice in plant nuclear genes and in cholorplasts. Chloroplasts differ from higher plants in that they encode only 30 tRNA species. Since chloroplasts have restricted their tRNA genes, the use of preferred codons by chloroplast-encoded proteins appears more extreme. However, a positive correlation has been reported between the level of isoaccepting tRNA for a given amino acid and the frequency with which this codon is used in the chloroplast genome (Pfitzinger et al. (1987) Nucl. Acids Res. 15:1377-1386. In general, the chloroplast codon profile more closely resembles that of unicellular organisms, with a strong bias towards the use of A+T in the degenerate third base.

Following are examples which illustrate procedures, including the best mode, for practicing the invention. These examples should not be construed as limiting. All percentages are by weight and all solvent mixture proportions are by volume unless otherwise noted.

### EXAMPLES

### Example 1 - Kinetics of C. sorokiniana Chloroplast Glutamate Dehydrogenases

The chloroplastic glutamate dehydrogenase α- and β-isoenzymes used in the following experiments are naturally produced by an organism characterized as *Chlorella sorokiniana.*

*C. sorokiniana* culture conditions. For kinetic characterization in both the aminating and deaminating directions, the α- and β-holoenzymes were purified from cells that were accumulating only one form of homohexameric GDH isoenzyme.

The *C. sorokiniana* cells (UTEX-1230, University of Texas algal culture collection; 3B2NA, Robert R. Schmidt, University of Florida, Microbiology Cell Science Department) were cultured autotrophically as previously described by Prunkard *et al., supra* in a modified basal salts medium. The modified medium contained in mM concentration: $CaCl_2$, 0.34; $K_2SO_4$, 6.0; $KH_2PO_4$, 18.4; $MgCl_2$, 1.5; in $\mu M$ concentration $CoCl_2$, 0.189; $CuCl_2$, 0.352; EDTA, 72; $FeCl_3$, 71.6; $H_3BO_3$, 38.8; $MnCl_2$, 10.1; $NH_4VO_4$, 0.20; $(NH_4)_6MO_7O_{24}$, 4.19; $NiCl_2$, 0.19; $SnCl_2$, 0.19; $ZnCl_2$, 0.734. The medium was supplemented with 1 mM $NH_4Cl$, 29 mM $NH_4Cl$, or 29 mM $KNO_3$ as a nitrogen source depending on the experimental conditions. The medium containing $NH_4Cl$ was adjusted to pH 7.4, and medium containing $KNO_3$ was adjusted to pH 6.8 with KOH after autoclaving. Cells were supplied with a 2% (v/v) $CO_2$-air mixture and light intensity sufficient to allow cell division into four progeny.

Purification of the NADP-GDH isoenzymes. For purification of the glutamate dehydrogenase α-isoenzyme, *C. sorokiniana* cells were cultured with continuous light in 29 mM ammonium medium in a 30 L Plexiglas chamber as previously described (Baker, A.L., R.R. Schmidt [1963] *Biochim. Biophys. Acta* 74:75-83). Cells were harvested at 4.0 $OD_{640}$ by centrifugation at 30,000 rpm through a Sharples centrifuge and washed two times in 10 mM Tris (pH 8.5 at 4°C). Pelleted cells (130 g) were stored at -20°C in 250 mL centrifuge bottles until use. Purification of NADP-GDH was accomplished using a modified procedure of Yeung *et al., supra.* Procedural modifications involved the substitution of Sephadex G-200 gel (Pharmacia) for G-150 gel in the gel-filtration column, and the addition of $NADP^+$ as a stabilizer to a final concentration of 0.1 mM to the gel-filtration buffer and all subsequent storage buffers. As a final modification, the $NADP^+$ affinity resin step was omitted and a preparative nondenaturing-PAGE step was substituted (Miller, P. W., W.D. Dunn, R.R. Schmidt [1994] *BioRad US/EG Bulletin 1897*).

The GDH deaminating enzyme assay solution was composed of 44 mM Tris, 20.4 mM glutamate, and 1.02 mM $NADP^+$, pH 8.8. The aminating assay solution was composed of 50 mM Tris, 25 mM $\alpha$-ketoglutarate, 0.357 mM NADPH, and 0.356 M $(NH_4)_2SO_4$, pH 7.4. One unit of enzyme activity was the amount of NADP-GDH required to reduce or to oxidize 1.0 $\mu$mol of $NADP^+$ or NADPH per minute at 38.5°C.

Sephadex G-200 column fractions possessing NADP-GDH activity were pooled and concentrated via Diaflow filtration. The soluble enzyme (68 mg) was protected from oxidation by the addition of DTT to a final concentration of 10 mM, and dialyzed for 30 minutes against 28.8 mM Tris, 192 mM glycine, 2 mM DTT (pH 8.4). The dialysate was clarified by centrifugation at 20,000g for 10 minutes at 4°C and was combined with 3 mL of 40% (w/v) sucrose and 1 mL of 0.02% bromophenol blue.

For preparative nondenaturing PAGE, a 3 cm tall 7% acrylamide (w/v, 28 acrylamide: 0.735 bis-acrylamide, pH 8.8) resolving gel, and a 2 cm tall 2% acrylamide (w/v, 1.6 acrylamide: 0.4 bis-acrylamide, pH 6.6) stacking gel were cast in the 28 mm ID gel tube of the Model 491 Prep Cell. All acrylamide stocks were pretreated with AG501-X8 mixed bed resin to remove any contaminating acrylic acid residue to prevent *in vitro* N-acylation of proteins during electrophoresis. The protein sample was electrophoresed at 15 mA constant power for 20 minutes and then for 3.5 hours at a constant power of 30 mA. Six milliliter fractions were collected and assayed for NADP-GDH deaminating activity and GDH containing fractions were pooled. The enzyme in the pooled fractions in 10 mM $KPO_4$ (pH 6.2), 0.1 mM $NADP^+$ was concentrated by Diaflow ultrafiltration to 1 mg/mL as determined by the method of Bradford, using BSA as a standard. The concentrated enzyme preparation was stored at -20°C. The purity of the preparation was determined by silver-staining to visualize proteins resolved by 10% (w/v) Tris-Tricine SDS-PAGE (Schagger, H., G. von Jagow [1987] *Anal. Biochem.* 166:368-379).

The NADP-GDH $\beta$-isoenryme was purified from a mixture of cells cultured for 240 minutes in 1 mM ammonium medium (14 g), 90 minutes in 1 mM ammonium medium (6 g), and for 20, 40, 60, and 80 minutes in 29 mM ammonium medium (1 g/time point) according to Bascomb and Schmidt, *supra.* The NADP-GDH $\beta$-isoenzyme was partially purified using a scaled down modified procedure of Yeung *et al., supra.* The DEAE sephacel ion exchange columns (pH 7.4, and pH 6) were scaled down to a 40 mL bed volume and a 400 mL linear KCl gradient (0 to 0.4 M) was used to clute the proteins in 3 mL fractions. The pH 6 DEAE ion-exchange column fractions containing NADP-GDH were combined into two pools; corresponding to the leading and trailing halves of the NADP-GDH activity peak. The separate pooled fractions were dialyzed against 10 mM $KPO_4$ (pH 6.2), 2 mM DTT for 16 hours, and affinity purified using Type 3 $NADP^+$ affinity gel (Pharmacia) as previously described (Bascomb and Schmidt, *supra*). The NADP-GDH in the pooled fractions was concentrated via Diaflow ultrafiltration to 2 mg/ml protein, as determined by the method of Bradford (Bradford, M.M. [1976] *Anal. Biochem.* 72:248-254), and stored at 4°C until further use. After resolution of the proteins by 8% (w/v) Tris-Tricine SDS-PAGE, the purity of the preparation was determined by silver staining.

Table 1 summarizes the $K_m$ values determined for both the $\alpha$- and $\beta$-homohexameric isoenzyme aminating reaction.

**Table 1**

| GDH Isoform | Substrate | $K_m$ Value (mM) |
| --- | --- | --- |
| $\alpha$-homohexamer | NADPH | 0.14 |
| | $NH_4^+$ | 0.02-3.5 |
| | $\alpha$-ketoglutarate | 0.35* |
| $\beta$-homohexamer | NADPH | 0.14 |
| | $NH_4^+$ | 77 |
| | $\alpha$-ketoglutarate | 12 |

*after Shatilov, V.R., W.L. Kretovich (1977) *Mol. Cell Biochem.* 15:201-212.

Table 2 summarizes the $K_m$ values determine for both the $\alpha$- and $\beta$-homohexameric isoenzyme deaminating reaction.

EP 0 859 849 B1

| Table 2 | | |
| --- | --- | --- |
| GDH Isoform | Substrate | $K_m$, Value (mM) |
| $\alpha$-homohexamer | NADP$^+$ | 0.04 |
| | Glutamate | 38.2 |
| $\beta$-homohexamer | NADP+ | 0.04 |
| | Glutamate | 32.3 |

Activity of the $\alpha$-,$\beta$-heterohexamer. The aminating and deaminating activities of the mixture of native NADP-GDH isoenzymes (heterohexamers composed of varying ratios of the $\alpha$- and $\beta$-subunits) were also measured with saturating levels of substrates throughout the 240 minute induction period (Fig. 1). The aminating and deaminating activities showed initial induction lags of 20 to 40 min, respectively. The aminating activity increased rapidly during the first 100 min, decreased sharply between 100 min and 140 min, and increased sharply once again between 140 min and 240 min. In contrast, the deaminating activity increased in almost a linear manner throughout the induction after the initial induction-lag.

During the 240 min induction period in 29 mM ammonium medium, the patterns of accumulation of the *Chlorella sorokiniana* NADP-GDH $\alpha$- and $\beta$-subunits in isoenzymes were also examined by use of a western blot immunodetection procedure following SDS polyacrylamide-gel electrophoresis (see Fig. 2). The NADP-GDH$\beta$-subunit was detected at $T_0$ and increased for the first 40 min followed by a gradual decrease through the remainder of the induction period. The $\alpha$-subunit was first detected at 20 min. This subunit accumulated at a low rate for the first 80 min, showed a marked increase between 80 min and 100 min, and thereafter accumulated in a linear manner at a lower rate for the remainder of the induction period. The transition from the $\beta$-subunit being the prominent species to the $\alpha$-subunit being prominent occurred between 60 and 80 min.

The aminating:deaminating activity ratio and the $\alpha$;$\beta$ subunit ratio were calculated to determine if changes in the subunit ratio in the mixture of NADP-GDH isoenzymes correlated with the predicted aminating:deaminating activity ratio during the time-course of the induction period (Table 3). Surprisingly, the highest aminating:deaminating ratio was observed at 60 min when the subunit ratio showed the $\beta$-subunit to be the prominent NADP-GDH antigen, whereas the $\alpha$-subunit was the prominent form when the aminating:deaminating activity ratio was the lowest. This latter result was not predictable in advance.

Prior to this discovery, substrate kinetic studies of purified $\alpha$- and $\beta$-homohexamers, the $\alpha$-homohexamer, with its very high affinity for ammonium (relative to the $\beta$-homohexamer), was assumed to be the isoenzyme-form with the highest aminating activity (i.e., biosynthetic capacity for glutamate synthesis). The results suggested that the individual subunits would act independently with respect to their kinetic properties in homo- and heterohexamers.

Comparison of the aminating:deaminating activity ratio with the $\alpha$:$\beta$ subunit ratio throughout the 240 min induction in 29 mM ammonium medium revealed an unexpected correlation between the maxima in these ratios (Table 3).

Table 3. NADP-GDH aminating :deaminating activity and $\alpha$-subunit:$\beta$-subunit ratios during ammonium induction period in *C. sorokiniana* cells.

| Table 3 | | |
| --- | --- | --- |
| Time (min) | Am:Deam Activity | $\alpha$:$\beta$ Subunit |
| 0 | 2.87 | 0.28 |
| 20 | 2.96 | 0.58 |
| 40 | 3.81 | 0.49 |
| 60 | 4.51 | 0.80 |
| 80 | 3.49 | 1.57 |
| 100 | 2.73 | 8.74 |
| 140 | 1.61 | 11.23 |
| 240 | 1.13 | 34.79 |

The peak in aminating:deaminating ratio occurred at 60 min at which time the $\beta$-subunit was the prominent but not exclusive antigen, whereas the $\alpha$-subunit was prominent when the aminating:deaminating ratio was lowest. Interestingly, the aminating activity was highest when both subunits were present, suggesting that heterohexamer(s), formed by combination(s) of the $\alpha$- and $\beta$-subunits, can have a higher aminating activity than a homohexamer. Based on the

much lower $K_m$ of the purified α-homohexamer that the β-homohexamer for ammonium, it had been predicted earlier that the α-homohexamer would have a higher aminating activity than any heterohexamer composed of the two subunits (Bascomb and Schmidt, 1987).

Example 2 - Sequencing of Polypeptides and Polynucleotides

Amino-terminal sequencing of the mature subunits. An aliquot of a preparation of purified NADP-GDH α-subunit (120 pmol) and a partially purified preparation of NADP-GDH α-subunit (80 pmol) and β-subunit (50 pmol) were resolved by 8% (w/v) Tris-Tricine SDS-PAGE and electroblotted to a PVDF membrane (Immobilon-P^SQ, Millipore) as described by Plough et al. (Plough, M., A.L. Jensen, V. Barkholt [1989] Anal. Biochem 181:33-39). To prevent in vitro acylation of the protein amino-terminal residues, all polyacrylamide solutions used in PAGE were treated with AG501-X8 mixed bed resin to remove contaminating acrylic acid. An Applied Biosystems, Inc. model 470A gas phase sequencer was utilized for automated Edman degradation amino sequence analysis. The PTH-aa derivatives were identified by RP-HPLC. Protein sequence analysis of the electroblotted proteins was provided by the Interdisciplinary Center for Biotechnology Research Protein Chemistry Core facility at the University of Florida.

The following N-terminal sequence was determined for the α-subunit: AVSLEEQISAMDATTGDFTA (SEQ ID NO. 5). The following N-terminal sequence was determined for the β-subunit: DATTGDFTAL (SEQ ID NO. 6). These sequences are identical to the ORF identified in the two NADP-GDH cDNAs and indicate the positions of the internal cleavage sites utilized to remove the chloroplast targeting peptide sequences. The chloroplast targeting peptide sequences (or chloroplast-transit peptides) can be useful for cell compartment localization with these and other amino acid sequences. The polynucleotides encoding the chloroplast-transit peptides can be used with other polynucleotide sequences to encode chloroplast-transit peptides.

cDNA isolation and sequencing. A pellet of C. sorokiniana cells stored at -70°C was resuspended 1 to 10 (w/v) in RNA breakage buffer: 0.1M Tris (pH8.5), 0.4M LiCl, 10 mM EGTA, 5 mM EDTA, 100 units/mL sodium heparin (Sigma, 100 units/mg), and 1 mM aurintricarboxylic acid (Sigma). The cell suspension was centrifuged at 7000g for 5 minutes at 4°C and the supernatant was discarded. The cell pellet was resuspended 1 to 10 (w/v) in RNA breakage buffer and ruptured by passage through a French pressure cell at 20,000 p.s.i. The cell homogenate was collected in a disposable 50 mL conical tube containing 0.05 times volume 20% (w/v) SDS, 0.05 times volume 0.5 M EDTA (pH 8), 200 μg/mL proteinase K, and allowed to incubate at room temperature for 15 minutes. One-half volume of TE buffer (Tris 10mM: EDTA 1mM, pH 8.0) equilibrated phenol was added to the homogenate and after a 3 minutes incubation a one-half volume of chloroform:isoamylalcohol (24:1,v/v) was added and mixed for 10 minutes on a wrist action shaker. The extracted homogenate was transferred to a 30 mL siliconized corex tube and centrifuged at 1000g for 10 minutes at 4°C. The upper aqueous phase was removed and repeatedly extracted with an equal volume of chloroform: isoamyl-alcohol (24:1, v/v), as described above, until the aqueous interface was clear. After the final extraction, the aqueous phase was combined with an equal volume of 2X LiCl-Urea buffer (4 M LiCl, 4 M urea, 2 mM EDTA, 1 mM aurintricarboxylic acid; Sigma) and the RNA was precipitated on ice for 16 hours at 4°C. The RNA precipitate was centrifuged at 4000g for 20 minutes at 4°C and the resulting pellet was rinsed once with 1X LiCl-Urea buffer and centrifuged again to pellet the RNA. The RNA pellet was solubilized in TE (pH 7.5) and an aliquot was quantified spectrophotometrically at 260 nm. After quantitation, the mRNA fraction was isolated from total cellular RNA using an oligo(dT) spin column kit. Poly (A)^+ RNA (50μg) from each preparation was combined and utilized for the commercial production of a custom λUni-ZAP XR C. sorokiniana cDNA library (Stratagene Cloning Systems, Palo Alto, CA).

The amplified λZAP library, containing 2 x 10^10 pfu/mL, was plated on twenty 150 mm petri plates at 50,000 pfu per plate for a total of 1 x 10^6 pfu screened. The phage plaques were absorbed to duplicate Hybond-N 132 mm circular membranes and treated according to the plaque blotting protocol of Amersham (1985, Amersham International plc, Arlington Heights, IL). Membranes were prehybridized in a common container in 200 mL of 2X PIPES (0.8 M NaCl, 20 mM PIPES, pH 6.5), 50% (w/v) formamide, 0.5% (w/v) SDS, 100 μg/mL denatured sheared salmon sperm DNA at 40°C. Blocked membranes were hybridized at 42°C in ten heat-sealable bags (four membranes/bag) in prehybridization buffer containing 1 x 10^6 cpm/membrane of a ^32P-labeled NADP-GDH 242 bp HCR cDNA probe on a lab rocker. The membranes were washed three times in 200 mL of 0.1X SSC, 0.1% (w/v) SDS for 20 minutes per wash at 50°C. Duplicate membranes were wrapped in plastic wrap and exposed to Kodak X-Omat AR film at -70°C for 28 hours. Putative NADP-GDH cDNA plaques, detected on duplicate membranes, were cored from the plate and plaque purified by secondary and tertiary screenings with the 242 bp conserved region probe. Putative NADP-GDH cDNA phage clones, selected in the primary screening, were combined and screened a second time with a ^32P-labeled 130 bp Eco RI/Bgl II cDNA fragment isolated from the 5' terminus of the most complete 5' end NADP-GDH cDNA clone. Ten plaque pure NADP-GDH clones were subcloned in pBluescript KS^+ (Stratagene) and transformed into E. coli DH5α F' (Bethesda Research Laboratories, BRL) via an in vivo excision protocol provided by Stratagene. All plasmid isolations were performed as described by Kraft et al., (Kraft, R, J. Tardiff, K.S. Krauter, L.A. Leinwand [1988] Biotechniques 6:544-547). Sequence analysis revealed all ten clones were identical at their 3'-termini and differed by varying degrees of truncation at their 5'-termini. The longest cDNA clone with a complete 3'-terminus designated pBGDc53 (SEQ ID NO. 7) was not long enough to encode either subunit; therefore, the 5'-terminal sequences were determined by RACE PCR.

The 5'-terminal NADP-GDH cDNA sequences were cloned using a modified anchored PCR procedure for the rapid amplification of cDNA ends (Frohman, M.A [1990] In D.H. Gelford, J.J. Snincky, TJ. White, eds, *PCR Protocols,* Academic Press, San Diego, CA, pp 28-38; Jain, R., R.H. Gorner, J.J. Murtagh [1992] *Biotechniques* 12:58-59). A mixture of poly(A)⁺ RNA, used in the synthesis of the λZAP library, was utilized to clone the 5' end of the NADP-GDH mRNA One hundred nanograms of the mRNA mixture were combined with 10 ng of a gene-specific primer (5'-CTCAAAGGCAAGGAACTTCATG-3', SEQ ID NO. 8), designed to hybridize to the conserved region of NADP-GDH mRNAs, heated for 5 minutes, and chilled on ice. First strand DNA synthesis was performed using Superscript™ reverse transcriptase (BRL) according to the supplier's protocol. The terminated reverse transcription reaction was treated with one unit of ribonuclease H for 20 minutes at 37°C, 5 minutes at 95°C, and extracted once with chloroform:isoamyl alcohol (24:1, v/v). Excess primers and dNTPs were removed by centrifugation at 2000 rpm through an Ultrafree-MC filterfuge tube (30,000 MW cutoff, Millipore) and the retentate was concentrated to 10 $\mu$l on a Savant Speedvac. The first-strand synthesis products were combined with 10 µL of tailing mix (1X tailing buffer [Promega Corp.], 0.4 mM dATP, 10 units terminal deoxytransferase) and incubated at 37°C for 10 minutes. The reaction mixture was heated to 95°C for 5 minutes, diluted to 0.5 mL with TE (pH 8), and utilized as a cDNA pool. A mixture of 5$\mu$L of the cDNA pool, 5 $\mu$L of Vent™ polymerase 10X buffer (New England Biolabs), 200 $\mu M$ of each dNTP, 25 pmol of a gene specific primer (SEQ ID NO. 8), 5 pmol of the poly(dT) adaptor primer (5'-GGGTCGACATTCTAGACAGAATTCGTGGATCC(T)$_{18}$-3'; SEQ ID NO. 9), 0.2 units Perfectmatch™ DNA polymerase enhancer (Stratagene), and 1 unit of Vent™ polymerase (NEB) in 50 $\mu$L was amplified according to Jain *et al., supra.* The PCR products were purified away from the excess primers by centrifugation at 2,000 rpm through an Ultrafree-MC unit. The retentate was collected and subjected to two more rounds of amplification using a new nested gene specific primer at each step (5'-GGACGAGTACTGCACGC-3', SEQ ID NO. 10; 5'-GATCTCGGTCAGCAGCTG-3', SEQ ID NO. 11, respectively) and an adaptor primer (5'-GGGTCGACATTCTAGACAGAA-3'; SEQ ID NO. 12). PCR amplifications were performed in a Model 480 thermocycler (Perkin-Elmer Cetus), and all custom oligonucleotides were synthesized by the ICBR DNA synthesis facility, University of Florida. The standard PCR reaction mixture consisted of 10 $\mu$L of 10X Vent™ polymerase buffer, 100 $\mu$M of each dNTP, 0.4 units of Perfectmatch™, 50 pmol of each primer, 1 unit Vent™ DNA polymerase in a 100 $\mu$l reaction volume. The 5' RACE-PCR products were gel purified, subcloned into the *Sma*I site of pUC 18, and transformed into *E. coli* DH5$\alpha$ for further characterization. RACE PCR identified two 5' cDNA clones, which overlapped with the previously identified pBODc 53 clone, that differed by a 42 nt insert identified in one clone designated pRGDc 60 (SEQ ID NO. 13) and lacking in the second cDNA designated pRGDc 61 (SEQ ID NO. 14).

Two additional cDNA clones lacking the RACE PCR polylinker, but possessing the complete 5'-termini corresponding to pRGDc 60 and 61 were constructed by RT-PCR amplification from mRNA using reaction conditions as described above and the gene specific primer pair (5'-CTTTCTGCTCGCCCTCTC-3', SEQ ID NO. 15, and SEQ ID NO. 11, above). The two PCR products were cloned into the *Sma*I site of pBluescript SK+ (Stratagene) and transformed into *E. coli* DH5$\alpha$ for further characterization. The cDNA clone that possessed the 42 nt insert was designated pGDc 63 (SEQ ID NO. 16) whereas the cDNA lacking the insert was designated pGDc 64 (SEQ ID NO. 17).

Full-length NADP-GDH cDNAs were constructed by restriction endonuclease treating pGDc 63 and 64 with *EcoR*I/*Apa*LI and gel purifying the resultant (264 bp; 222 bp, respectively) fragments. The gel purified fragments were ligated to a purified *Apa*LI/*Xho*I restriction fragment of pBGDc 53 and the full length ligation products (SEQ ID NO. 18; SEQ ID NO. 19) were gel agarose gel purified and utilized in subsequent PCR reactions.

<u>Expression of $\alpha$- and $\beta$-homohexamers in *E. coli*.</u> Using the gel purified product (SEQ ID NO. 18), PCR mutagenesis was performed to remove the chloroplast targeting signal from the full-length cDNA and yield cDNAs encoding specifically the mature $\alpha$- and $\beta$-subunits. Two sets of primer pairs were designed to synthesize $\alpha$- and $\beta$-GDH subunit genes.

The following primer was designed to add a methionine to the amino terminus of the processed mature $\alpha$-NADP-GDH subunit (alanine-41) to allow translation initiation and to generate a 5' *Nde*I site for subcloning purposes: 5'-CATATGGCCGTCTCGCTGGAGGAG-3' (SEQ ID NO. 20). The following second primer was designed to hybridize to the 3' terminus of the template DNA at a position 20 nt 3' of the endogenous TAA termination codon: 5'-GTTGGATTGCCGGTGAGCC-3' (SEQ ID NO. 21).

The following primer was designed to add a methionine to the amino terminus of the processed mature $\beta$-subunit (aspartate-38) to allow translation initiation and to generate a 5' *Nde*I site for subcloning purposes: 5'-CATATGGACGCCACCACCGGC-3' (SEQ ID NO. 22). The second 3' primer used in the PCR amplification was the 3'-terminus primer (SEQ ID NO. 21) described for the $\alpha$-subunit amplification.

PCR cycling conditions were as follows: 95°C, 50 seconds; 64°C, 1 minute; 72°C,1 minute 35 seconds (30 cycles). Primer, dNTP, Vent polymerase, and other reaction component concentrations were as previously described. The 1506 bp $\alpha$-NADP-GDH subunit gene (SEQ ID NO. 23) and 1473 bp$\beta$-GDH subunit gene (SEQ ID NO. 25) PCR products were gel purified and given a 3' adenine nucleotide overhang by incubating the purified fragment with 100 $\mu$M

dATP and *Taq* polymerase for 15 minutes at 72°C. The modified PCR products were cloned into the PCRII T/A cloning vector (Invitrogen) and transformed into competent *E. coli* cells. Clones bearing the inserts were selected by blue-white screening, plasmid purified, and digested with *Nde*I/*Bam*HI to select for the proper orientation in the cloning vector. The selected plasmids were restricted with *Nde*I and *Bam*HI (*Bam*HI site provided by vector) and directionally cloned under the control of the IPTG inducible T7 polymerase promoter of pET 11a and pET 15b bacterial expression vectors (Novagen) linearized with *Nde*I/*Bam*HI, and transformed into DH5α. Transformants were screened by *Nde*I/*Bam*HI restriction analysis and clones possessing the properly oriented α- and β-subunit cDNAs (SEQ ID NO. 23; SEQ ID NO. 25) were selected, plasmid purified, and transformed into *E. coli* BL21(DE3) for protein expression purposes.

*E. coli* BL21(DE3) cells transformed with pET 11a-α-cDNA and pET 11a-β-cDNA constructs were induced with 100 mM IPTG for 1 hour. Protein extracts from the induced cells were tested by enzyme analysis for NADP-GDH activity, and the denatured proteins were resolved by SDS gel electrophoresis, and visualized by coomassie staining. The proteins expressed by the mature α-subunit cDNA (SEQ ID NO. 23) and the β-subunit cDNA (SEQ ID NO. 25) have the amino acid sequences shown in SEQ ID NO. 24 (α-subunit) and SEQ ID NO. 26 (β-subunit). The recombinant GDH subunits were verified by crossreactivity with rabbit *anti-Chlorella* NADP-GDH antibodies.

Under conditions not optimized for maximal induction, the *E. coli* cells, possessing the α- and β-GDH cDNAs and induced with IPTG, showed 60- and 7,000-fold increases in NADP-GDH activity relative to uninduced controls, respectively. The recombinant α- and β-NADP-GDHs are currently being analyzed to verify kinetic and biochemical properties.

The over-expression and assembly of the *C. sorokiniana* chloroplastic GDHs into active enzymes provides proof that the DNA constructs engineered via PCR are transcribed and translated into authentic proteins. The aforementioned constructs were then utilized for cytosolic expression of the algal GDHs in transgenic plants.

<u>Transformation of plants.</u> A method for producing genetically transformed plants that express increased levels of a specific GDH requires the introduction of a double-stranded recombinant DNA molecule into the nuclear genome of a plant cell. The DNA molecule must (1) contain a structural DNA for the GDH enzyme being introduced into the plant cell; (2) possess a promoter which functions in plants to regulate the production of an RNA sequence in a constitutive or tissue-specific manner by RNA polymerase enzyme; and (3) have a 3'-untranslated region which functions to cause transcriptional termination and the addition of polyadenylated nucleotides to the 3' end of the RNA. The resulting primary RNA molecule is subsequently processed in the nucleus, a process which involves the removal of intronic sequences and the addition of polyadenylate nucleotides to the 3' end of the mRNA.

Promoters which are useful in the present invention are those that can initiate transcription in a constitutive manner or in a tissue-specific manner where glutamate production or catabolism is desired. An example of a useful constitutive promoter is the CaMV enhanced 35S promoter that directs the synthesis of RNA in a tissue independent manner. Promoters which cause production of GDH specifically in seeds, stems, roots, leaves, or specific cell types in these tissues are useful in the present invention. For example, the seed-specific Phaseolin promoter is one such tissue-specific promoter. Thus native promoters for maize, wheat, barley, and rice may be obtained and used in the present invention as well as heterologous promoters from other organisms shown to function in a constitutive/tissue-specific manner.

<u>Introns.</u> Generally, optimal expression in monocotyledonous plants is obtained when an intron sequence is inserted between the promoter sequence and the structural gene sequence. An example of such an intron sequence is the HSP 70 intron described in WO 93/19189.

<u>Polyadenylation signal.</u> The DNA constructs of the present invention can possess a 3' untranslated region which functions in plants to direct the addition of polyadenylate nucleotides to the 3' end of the RNA. An example of a suitable 3' untranslated region is the polyadenylation signal of the *Agrobacterium* tumor inducing plasmid, i.e., nopaline synthatase (NOS) gene.

<u>Plastid targeting sequence.</u> The DNA constructs of the present invention can optionally contain a plastid targeting sequence. The plastid targeting sequence directs the import of the protein into the plastid, and is removed during importation. The plastid targeting sequence can be, but is not limited to, the native chloroplast targeting peptide (CTP) identified in the *C. sorokiniana* NADP-GDH full-length cDNAs which encode the precursor proteins. A fusion of a selected plastid targeting sequence and the mature α- and β-NADP-GDH subunit sequences can be made by standard procedures and used in the present invention. GDH subunits lacking these targeting sequences are typically found in the cytoplasm of the cell. Such a cytosolic localized enzyme can be useful in capturing ammonium or glutamate compartmentalized in the cytosol of the cell.

<u>GDH gene sources.</u> The GDH gene used in the DNA constructs of the present invention can be any GDH gene. It is not limited to the *C. sorokiniana* GDH genes described above, although they are preferred. For example, a GDH gene from bacteria or fungi can be used. The examples provided use the α- and β-GDH genes of *C. sorokiniana,* but should not be interpreted in any way to limit the scope of the present invention. Individuals skilled in the art will recognize that various other genes as well as alterations can be made to genes and methods described herein while not departing from the spirit and scope of the present invention. For example, mutagenesis and routine screening can be implemented by techniques well known in the art to produce mutant variants that lack regulation by the cofactor NADPH.

Transient expression in maize protoplasts. In order to test the expression of the *C. sorokiniana* GDH subunits and their assembly into active enzymes in *Zea mays* cells, vectors were constructed to contain the CaMV E35S promoter, the coding sequence for the mature α-subunit (pMON21904) or β- subunit (pMON21905), the NOS 3'-untranslated polyadenylation region, and kanamycin resistance for selection in *E. coli.* The α- and β-subunit genes were isolated as a *Xba*l-EcoRI fragment from pET 11a-α-cDNA and pET 11a-β-cDNA, respectively. The GDH genes were ligated into the *Xba*l-*EcoR*l E35S promoter, NOS 3', kanamycin resistance bearing region of pMON22072 to give pMON21904, and pMON21905. The DNA constructs were electroporated into maize and wheat protoplast according to the method of Sheen *et al.* (*The Plant Cell* Vol. 3, 225-245).

Analysis of transformed maize protoplasts. Pelleted protoplast samples transformed with pMON21904 (α-subunit), pMON21905 (β-subunit), pMON21709 (kanamycin negative control DNA), and no DNA were thawed in 0.2 mL of GDH cell breakage buffer (Yeung *et al., supra*) on ice. The cells in each suspension were homogenized twice for 30 seconds, chilled on ice, and clarified at 14,000 rpm for 10 minutes. Cell extracts were assayed in the deaminating direction at 38.5°C according to Yeung *et al., supra.* Total protein content of the cell extracts was determined using the BioRad microprotein assay according to the manufacturer's protocol. Activities were normalized against total protein content for comparisons among different preparations. One unit of GDH activity is defined as the amount of enzyme necessary to reduce 1 $\mu$mol of NADP per minute at 38.5°C.

Protoplasts transformed with the control vector pMON21709 (n=3) or protoplasts not transformed (n=3) had no detectable NADP-GDH activity. Protoplasts transformed with pMON21904 (n=3) expressed 3-31 Units mg$^{-1}$ protein of GDH activity, whereas pMON21905 transformed protoplasts (n=3) 1.96 Units mg$^{-1}$ protein.

The high level of activity observed for the protoplasts transformed with the cytoplasmic expressed *C. sorokiniana* α- and β-NADP-GDH genes provides evidence that the GDH subunits are expressed in heterologous plant systems. Additionally, expression levels demonstrate that the subunits are assembled into active enzymes. Generally, it would be readily apparent to persons of ordinary skill in the art that superfluous sequences added to the described sequences, or fragments of the nucleotide or amino acid sequences described herein, which result in polynucleotides or amino acid sequences that function similarly or equivalently to the sequences expressly described herein, should also be considered part of this invention. They can easily and routinely be produced by techniques well known in the art, for example, by time-controlled *Bal*31 exonuclease digestion of the full-length DNA, followed by expression of the resulting fragments and routine screening of the expression products as described in the foregoing example. In addition, it would be readily accepted by ordinarily skilled artisans that the function, property, or utility of the described sequences can be negatived by inserting mutations into the sequences by standard techniques and procedures. These mutations which, by implication, effectively serve to remove the property or function inherent in the sequences as described are hereby expressly included as part of the invention. For example, a clear distinction between the α- and β-subunits of the *C. sorokiniana* is the 11-amino acid polypeptide sequence at the N-terminus of the α-subunit, but absent in the β-subunit. This sequence can affect the affinity, specificity, and modulation of ammonium compounds by the enzyme. Therefore, it would be apparent that inserting (if absent) or removing (if present) the appropriate sequence, or its functional equivalent, to effect a difference in certain characteristics of other GDH genes, or their products, would be easily carried out by those persons.

**Claims**

1. A polynucleotide comprising a nucleotide sequence encoding an amino acid sequence selected from SEQ ID NO: 2, SEQ ID NO:4, SEQ ID NO: 24 and SEQ ID NO:26, or a fragment thereof having glutamate dehydrogenase (GDH) activity.

2. A polynucleotide comprising a nucleotide sequence selected from SEQ ID NO:1, SEQ ID NO: 3, SEQ ID NO:18, SEQ ID NO: 19, SEQ ID NO: 23 and SEQ ID NO: 25, or a fragment thereof encoding a peptide having GDH activity.

3. The polynucleotide according to claim 2, comprising a nucleotide sequence selected from SEQ ID NO: 1, SEQ ID NO: 3, SEQ ID NO: 23 and SEQ ID NO:25, or a fragment thereof encoding a peptide having GDH activity.

4. The polynucleotide according to claim 2, forming a chimeric gene capable of expression in plant cells, said polynucleotide being operably linked to a plant-expressible promoter and a plant polyadenylation sequence.

5. The polynucleotide according to claim 2, comprising at least one nucleotide sequence encoding an NADP-GDH alpha-subunit and at least one nucleotide sequence encoding an NADP-GDH beta-subunit.

6. The polynucleotide according to claim 5, wherein said polynucleotide further comprises a promoter for said polynucleotide encoding an NADP-GDH alpha-subunit and an NADP-GDH beta-subunit.

7. The polynucleotide according to claim 6, which comprises different promoters for the alpha-subunit and the beta-subunit.

8. The polynucleotide according to claim 6, wherein said promoter has differential activity for the alpha and beta-subunits.

9. The polynucleotide according to claim 5, comprising a nucleotide sequence selected from SEQ ID NO: 1 and SEQ ID NO: 23 and a nucleotide sequence selected from SEQ ID NO: 3 and SEQ ID NO: 25.

10. A transformed host cell comprising a polynucleotide according to claim 1.

11. The transformed host cell according to claim 10, wherein said polynucleotide comprises a nucleotide sequence selected from SEQ ID NO:1, SEQ ID NO: 3, SEQ ID NO:23 and SEQ ID NO:25.

12. A method for modulating nitrogen metabolism in a plant cell, which comprises transforming said plant cell to comprise a polynucleotide according to claim 1, and maintaining said cell or descendants thereof under conditions wherein said polypeptide is expressed.

13. The method according to claim 12, wherein said polynucleotide has a nucleotide sequence encoding at least one NADP-GDH alpha-subunit and at least one nucleotide sequence encoding an NADP-GDH beta-subunit.

14. The method according to claim 13, wherein said polynucleotide encodes a polypeptide comprising an amino acid sequence selected from SEQ ID NO:2 and SEQ ID NO:24 and an amino acid sequence selected from SEQ ID NO: 4 and SEQ ID NO:26.

15. The method according to any of claims 12 to 14, wherein said polynucleotide is operably linked to a plant expressible promoter and to a plant polyadenylation sequence.

16. The method according to any of claims 12 to 15, whereby the assimilation of inorganic nitrogen into organic nitrogen is increased.

17. The method according to any of claims 12 to 16, whereby crop yield is increased, or ammonium assimilation, osmotic stress tolerance or composition of the plant is altered.

18. A polypeptide comprising an amino acid sequence selected from SEQ ID NO:2, SEQ ID NO: 4, SEQ ID NO: 5, SEQ ID NO: 6, SEQ ID NO: 24 and SEQ ID NO: 26, or a fragment thereof having GDH activity.

19. A polypeptide comprising at least one NADP-GDH alpha-subunit that comprises an amino acid sequence selected from SEQ ID NO:2 and SEQ ID NO:24, or a fragment thereof having GDH activity, and at least one NADP-GDH beta-subunit.

20. The polypeptide according to claim 19, wherein the beta-subunit comprises an amino acid sequence selected from SEQ ID NO:4 and SEQ ID NO:26.

**Patentansprüche**

1. Polynucleotid, umfassend eine Nucleotidsequenz, die für eine Aminosäuresequenz, die aus SEQ ID NO:2, SEQ ID NO:4, SEQ ID NO:24 und SEQ ID NO:26 ausgewählt ist, oder für ein Fragment davon mit Glutamat-Dehydrogenase (GDH)-Aktivität kodiert.

2. Polynucleotid, umfassend eine Nucleotidsequenz, die aus SEQ ID NO:1, SEQ ID NO:3, SEQ ID NO:18, SEQ ID NO:19, SEQ ID NO:23 und SEQ ID NO:25 ausgewählt ist, oder ein Fragment davon, das für ein Peptid mit GDH-Aktivität kodiert.

3. Polynucleotid nach Anspruch 2, umfassend eine Nucleotidsequenz, die aus SEQ ID NO:1, SEQ ID NO:3, SEQ ID NO:23 und SEQ ID NO:25 ausgewählt ist, oder ein Fragment davon, das für ein Peptid mit GDH-Aktivität kodiert.

4. Polynucleotid nach Anspruch 2, das ein chimäres Gen bildet, das zur Expression in Pflanzenzellen befähigt ist, wobei das Polynucleotid funktionell mit einem exprimierbaren Pflanzen-Promotor und einer Pflanzen-Polyadenylierungssequenz verknüpft ist.

5. Polynucleotid nach Anspruch 2, umfassend mindestens eine Nucleotidsequenz, die für eine NADP-GDH-alpha-Untereinheit kodiert, und mindestens eine Nucleotidsequenz, die für eine NADP-GDH-beta-Untereinheit kodiert.

6. Polynucleotid nach Anspruch 5, wobei das Polynucleotid ferner einen Promotor für das Polynucleotid, das für eine NADP-GDH-alpha-Untereinheit und eine NADP-GDH-beta-Untereinheit kodiert, umfasst.

7. Polynucleotid nach Anspruch 6, das verschiedene Promotoren für die alpha-Untereinheit und die beta-Untereinheit umfasst.

8. Polynucleotid nach Anspruch 6, wobei der Promotor eine unterschiedliche Aktivität für die alpha- und beta-Untereinheiten aufweist.

9. Polynucleotid nach Anspruch 5, umfassend eine Nucleotidsequenz, die aus SEQ ID NO:1 und SEQ ID NO:23 ausgewählt ist, und eine Nucleotidsequenz, die aus SEQ ID NO:3 und SEQ ID NO:25 ausgewählt ist.

10. Transformierte Wirtszelle, umfassend ein Polynucleotid nach Anspruch 1.

11. Transformierte Wirtszelle nach Anspruch 10, wobei das Polynucleotid eine Nucleotidsequenz, die aus SEQ ID NO: 1, SEQ ID NO:3, SEQ ID NO:23 und SEQ ID NO:25 ausgewählt ist, umfasst.

12. Verfahren zum Modulieren des Stickstoff-Stoffwechsels in einer Pflanzenzelle, umfassend das Transformieren der Pflanzenzelle in der Weise, dass sie ein Polynucleotid nach Anspruch 1 umfasst, und das Halten der Zelle oder von Abkömmlingen davon unter Bedingungen, bei denen das Polypeptid exprimiert wird.

13. Verfahren nach Anspruch 12, wobei das Polynucleotid eine Nucleotidsequenz, die für mindestens eine NADP-GDH-alpha-Untereinheit kodiert, und mindestens eine Nucleotidsequenz, die für eine NADP-GDH-beta-Untereinheit kodiert, aufweist.

14. Verfahren nach Anspruch 13, wobei das Polynucleotid für ein Polypeptid kodiert, das für eine Aminosäuresequenz, die aus SEQ ID NO:2 und SEQ ID NO:24 ausgewählt ist, und für eine Aminosäuresequenz, die aus SEQ ID NO:4 und SEQ ID NO:26 ausgewählt ist, kodiert.

15. Verfahren nach einem der Ansprüche 12 bis 14, wobei das Polynucleotid funktionell mit einem exprimierbaren Pflanzen-Promotor und einer Pflanzen-Polyadenylierungssequenz verknüpft ist.

16. Verfahren nach einem der Ansprüche 12 bis 15, wobei die Assimilation von anorganischem Stickstoff zu organischem Stickstoff verstärkt wird.

17. Verfahren nach einem der Ansprüche 12 bis 16, wobei die Ernteausbeute erhöht wird oder die Ammonium-Assimilation, die osmotische Stresstoleranz oder die Zusammensetzung der Pflanze verändert werden.

18. Polypeptid, umfassend eine Aminosäuresequenz, die aus SEQ ID NO:2, SEQ ID NO:4, SEQ ID NO:5, SEQ ID NO:6, SEQ ID NO:24 und SEQ ID NO:26 ausgewählt ist, oder ein Fragment davon mit GDH-Aktivität.

19. Polypeptid, umfassend mindestens eine NADP-GDH-alpha-Untereinheit, die eine Aminosäuresequenz, die aus SEQ ID NO:2 und SEQ ID NO:24 ausgewählt ist, oder ein Fragment davon mit GDH-Aktivität und mindestens eine NADP-GDH-beta-Untereinheit umfasst.

20. Polypeptid nach Anspruch 19, wobei die beta-Untereinheit eine Aminosäuresequenz umfasst, die aus SEQ ID NO: 4 und SEQ ID NO:26 ausgewählt ist.

**Revendications**

1. Polynucléotide comprenant une séquence de nucléotides codant pour une séquence d'acides aminés choisie parmi SEQ ID NO:2, SEQ ID NO: 4, SEQ ID NO: 24 et SEQ ID NO: 26, ou un fragment de celles-ci présentant une activité de glutamate déshydrogénase (GDH).

2. Polynucléotide comprenant une séquence de nucléotides choisie parmi SEQ ID NO: 1, SEQ ID NO: 3, SEQ ID NO: 18, SEQ ID NO: 19, SEQ ID NO: 23, et SEQ ID NO: 25, ou un fragment de celles-ci codant pour un peptide présentant une activité GDH.

3. Polynucléotide selon la revendication 2, comprenant une séquence de nucléotides choisie parmi SEQ ID NO: 1, SEQ ID NO: 3, SEQ ID NO: 23, et SEQ ID NO: 25, ou un fragment de celles-ci codant pour un peptide présentant une activité GDH.

4. Polynucléotide selon la revendication 2, formant un gène chimérique capable d'expression dans des cellules végétales, ledit polynucléotide étant lié de manière fonctionnelle à un promoteur exprimable par la plante et à une séquence de polyadénylation de la plante.

5. Polynucléotide selon la revendication 2, comprenant au moins une séquence de nucléotides codant pour une sousunité alpha de NADP-GDH et au moins une séquence de nucléotides codant pour une sous-unité bêta de NADP-GDH.

6. Polynucléotide selon la revendication 5, dans lequel ledit polynucléotide comprend en outre un promoteur pour ledit polynucléotide codant pour une sous-unité alpha de NADP-GDH et une sous-unité bêta de NADP-GDH.

7. Polynucléotide selon la revendication 6, qui comprend des promoteurs différents pour la sous-unité alpha et la sousunité bêta.

8. Polynucléotide selon la revendication 6, dans lequel ledit promoteur présente une activité différencielle pour les sous-unités alpha et bêta.

9. Polynucléotide selon la revendication 5, comprenant une séquence de nucléotides choisie parmi SEQ ID NO: 1 et SEQ ID NO: 23 et une séquence de nucléotides choisie parmi SEQ ID NO: 3 et SEQ ID NO: 25,

10. Cellule hôte transformée comprenant un polynucléotide selon la revendication 1.

11. Cellule hôte transformée selon la revendication 10, dans laquelle le polynucléotide comprend une séquence de nucléotides choisies parmi SEQ ID NO: 1, SEQ ID NO: 3, SEQ ID NO: 23, et SEQ ID NO: 25.

12. Procédé de modulation du métabolisme de l'azote dans une cellule végétale, qui consiste à transformer ladite cellule végétale pour qu'elle comprenne un polynucléotide selon la revendication 1, et à maintenir ladite cellule ou ses descendants dans des conditions dans lesquelles ledit polypeptide s'exprime.

13. Procédé selon la revendication 12, dans lequel ledit polynucléotide possède une séquence de nucléotides codant pour au moins une sous-unité alpha de NADP-GDH et au moins une séquence de nucléotides codant pour une sous-unité bêta de NADP-GDH.

14. Procédé selon la revendication 13, dans lequel ledit polynucléotide code un polypeptide comprenant une séquence d'acides aminés choisie parmi SEQ ID NO: 2 et SEQ ID NO: 24 et une séquence d'acides aminés choisie parmi SEQ ID NO: 4 et SEQ ID NO: 26.

15. Procédé selon l'une quelconque des revendications 12 à 14, dans lequel ledit polynucléotide est lié de manière fonctionnelle à un promoteur exprimable par la plante et à une séquence de polyadénylation de la plante.

16. Procédé selon l'une quelconque des revendications 12 à 15, dans lequel l'assimilation d'azote minéral en azote organique est accrue.

17. Procédé selon l'une quelconque des revendications 12 à 16, dans lequel le rendement de récolte est accru, ou l'assimilation en ammonium, la tolérance au stress osmotique ou la composition de la plante est modifiée.

18. Polypeptide comprenant une séquence d'acides aminés choisie parmi SEQ ID NO:2, SEQ ID NO: 4, SEQ ID NO: 5, SEQ ID NO: 6, SEQ ID NO: 24 et SEQ ID NO: 26, ou un fragment de celles-ci présentant une activité de GDH.

19. Polypeptide comprenant au moins une sous-unité alpha de NADP-GDH qui comprend une séquence d'acides aminés choisie parmi SEQ ID NO: 2 et SEQ ID NO: 24 ou un fragment de celles-ci présentant une activité de GDH, et au moins une sous-unité bêta de NADP-GDH.

20. Polypeptide selon la revendication 19, dans lequel la sous-unité bêta comprend une séquence d'acides aminés choisie parmi SEQ ID NO: 4 et SEQ ID NO: 26.

FIG. 1

FIG. 2

SEQUENCE LISTING

    (1) GENERAL INFORMATION:

        (i) APPLICANT INFORMATION:

            Applicant Name(s): University of Florida
            Street Address: 223 Grinter Hall
            City: Gainesville
            State/Province: Florida
            Country: US
            Postal code/Zip: 32611
            Phone number: (352) 392-8929 Fax: (352) 392-6600

        (ii) TITLE OF INVENTION: NOVEL POLYPEPTIDES AND POLYNUCLEOTIDES RELATING TO THE α- AND β-SUBUNITS OF GLUTAMATE DEHYDROGENASES AND METHODS OF USE

        (iii) NUMBER OF SEQUENCES: 26

(iv) CORRESPONDENCE ADDRESS:

    (A) ADDRESSEE: Saliwanchik & Saliwanchik
    (B) STREET: 2421 N.W. 41st Street, Suite A-1
    (C) CITY: Gainesville
    (D) STATE: Florida
    (E) COUNTRY: USA
    (F) ZIP: 32606

(v) COMPUTER READABLE FORM:

    (A) MEDIUM TYPE: Floppy disk
    (B) COMPUTER: IBM PC compatible
    (C) OPERATING SYSTEM: PC-DOS/MS-DOS
    (D) SOFTWARE: Patent In Release #1.0, Version #1.25

(vi) CURRENT APPLICATION DATA:

    (A) APPLICATION NUMBER: US
    (B) FILING DATE:
    (C) CLASSIFICATION:

(viii) ATTORNEY/AGENT INFORMATION:

    (A) NAME: Whitlock, Ted W.
    (B) REGISTRATION NUMBER: 36,965
    (C) REFERENCE/DOCKET NUMBER: UF155

(ix) TELECOMMUNICATION INFORMATION:

    (A) TELEPHONE: (904) 375-8100
    (B) TELEPAX: (904) 372-5800

(2) INFORMATION FOR SEQ ID NO:1:

(i) SEQUENCE CHARACTERISTICS:

    (A) LENGTH: 2140 base pairs
    (B) TYPE: nucleic acid
    (C) STRANDEDNESS: double
    (D) TOPOLOGY: linear

(ii) MOLECULE TYPE: cDNA

(ix) FEATURE:

    (A) NAME/KEY: CDS
    (B) LOCATION: 33..1610

(xi) SEQUENCE DESCRIPTION: SEQ ID NO:1:

```
CTCCTTTCTG CTCGCCCTCT CTCCGTCCCG CC ATG CAG ACC GCC CTC GTC GCC
53
                                      Met Gln Thr Ala Leu Val Ala
                                       1                 5

AAG CCT ATC GTG GCC GCC CCG CTG GCG GCA CGC CCG CGC TGC CTC GCG
101
Lys Pro Ile Val Ala Ala Pro Leu Ala Ala Arg Pro Arg Cys Leu Ala
             10              15              20

CCG TGG CCG TGC GCG TGG GTC CGC TCC GCC AAG CGC GAT GTC CGC GCC
149
Pro Trp Pro Cys Ala Trp Val Arg Ser Ala Lys Arg Asp Val Arg Ala
         25              30              35

AAG GCC GTC TCG CTG GAG GAG CAG ATC TCC GCG ATG GAC GCC ACC ACC
197
Lys Ala Val Ser Leu Glu Glu Gln Ile Ser Ala Met Asp Ala Thr Thr
 40              45              50                      55

GGC GAC TTC ACG GCG CTG CAG AAG GCG GTG AAG CAG ATG GCC ACC AAG
245
Gly Asp Phe Thr Ala Leu Gln Lys Ala Val Lys Gln Met Ala Thr Lys
                 60              65                      70

GCG GGC ACT GAG GGC CTG GTG CAC GGC ATC AAG AAC CCC GAC GTG CGC
293
Ala Gly Thr Glu Gly Leu Val His Gly Ile Lys Asn Pro Asp Val Arg
                 75              80                      85

CAG CTG CTG ACC GAG ATC TTC ATG AAG GAC CCG GAG CAG CAG GAG TTC
341
Gln Leu Leu Thr Glu Ile Phe Met Lys Asp Pro Glu Gln Gln Glu Phe
                 90              95              100

ATG CAG GCG GTG CGC GAG GTG GCC GTC TCC CTG CAG CCC GTG TTC GAG
389
Met Gln Ala Val Arg Glu Val Ala Val Ser Leu Gln Pro Val Phe Glu
                105             110             115

AAG CGC CCC GAG CTG CTG CCC ATC TTC AAG CAG ATC GTT GAG CCT GAG
437
Lys Arg Pro Glu Leu Leu Pro Ile Phe Lys Gln Ile Val Glu Pro Glu
120             125             130                     135

CGC GTG ATC ACC TTC CGC GTG TCC TGG CTG GAC GAC GCC GGC AAC CTG
485
Arg Val Ile Thr Phe Arg Val Ser Trp Leu Asp Asp Ala Gly Asn Leu
                140             145             150

CAG GTC AAC CGC GGC TTC CGC GTG CAG TAC TCG TCC GCC ATC GGC CCC
533
Gln Val Asn Arg Gly Phe Arg Val Gln Tyr Ser Ser Ala Ile Gly Pro
                155             160             165

TAC AAG GGC GGC CTG CGC TTC CAC CCC TCC GTG AAC CTG TCC ATC ATG
581
Tyr Lys Gly Gly Leu Arg Phe His Pro Ser Val Asn Leu Ser Ile Met
                170             175             180

AAG TTC CTT GCC TTT GAG CAG ATC TTC AAG AAC AGC CTG ACC ACC CTG
629
Lys Phe Leu Ala Phe Glu Gln Ile Phe Lys Asn Ser Leu Thr Thr Leu
                185             190             195

CCC ATG GGC GGC GGC AAG GGC GGC TCC GAC TTC GAC CCC AAG GGC AAG
677
```

```
Pro Met Gly Gly Gly Lys Gly Gly Ser Asp Phe Asp Pro Lys Gly Lys
200           205           210           215


AGC GAC GCG GAG GTG ATG CGC TTC TGC CAG TCC TTC ATG ACC GAG CTG
725
Ser Asp Ala Glu Val Met Arg Phe Cys Gln Ser Phe Met Thr Glu Leu
              220           225           230


CAG CGC CAC ATC AGC TAC GTG CAG GAC GTG CCC GCC GGC GAC ATC GGC
773
Gln Arg His Ile Ser Tyr Val Gln Asp Val Pro Ala Gly Asp Ile Gly
              235           240           245


GTG GGC GCG CGC GAG ATT GGC TAC CTT TTC GGC CAG TAC AAG CGC ATC
821
Val Gly Ala Arg Glu Ile Gly Tyr Leu Phe Gly Gln Tyr Lys Arg Ile
              250           255           260


ACC AAG AAC TAC ACC GGC GTG CTG ACC CCG AAG GGC CAG GAG TAT GGC
869
Thr Lys Asn Tyr Thr Gly Val Leu Thr Pro Lys Gly Gln Glu Tyr Gly
              265           270           275


GGC TCC GAG ATC CGC CCC GAG GCC ACC GGC TAC GGC GCC GTG CTG TTT
917
Gly Ser Glu Ile Arg Pro Glu Ala Thr Gly Tyr Gly Ala Val Leu Phe
280           285           290           295


GTG GAG AAC GTG CTG AAG GAC AAG GGC GAG AGC CTC AAG GGC AAG CGC
965
Val Glu Asn Val Leu Lys Asp Lys Gly Glu Ser Leu Lys Gly Lys Arg
              300           305           310


TGC CTG GTG TCT GGC GCG GGC AAC GTG GCC CAG TAC TGC GCG GAG CTG
1013
Cys Leu Val Ser Gly Ala Gly Asn Val Ala Gln Tyr Cys Ala Glu Leu
              315           320           325


CTG CTG GAG AAG GGC GCC ATC GTG CTG TCG CTG TCC GAC TCC CAG GGC
1061
Leu Leu Glu Lys Gly Ala Ile Val Leu Ser Leu Ser Asp Ser Gln Gly
              330           335           340


TAC GTG TAC GAG CCC AAC GGC TTC ACG CGC GAG CAG CTG CAG GCG GTG
1109
Tyr Val Tyr Glu Pro Asn Gly Phe Thr Arg Glu Gln Leu Gln Ala Val
              345           350           355


CAG GAC ATG AAG AAG AAG AAC AAC AGC GCC CGC ATC TCC GAG TAC AAG
1157
Gln Asp Met Lys Lys Lys Asn Asn Ser Ala Arg Ile Ser Glu Tyr Lys
360           365           370           375


AGC GAC ACC GCC GTG TAT GTG GGC GAC CGC CGC AAG CCT TGG GAG CTG
1205
Ser Asp Thr Ala Val Tyr Val Gly Asp Arg Arg Lys Pro Trp Glu Leu
              380           385           390


GAC TGC CAG GTG GAC ATC GCC TTC CCC TGC GCC ACC CAG AAC GAG ATC
1253
Asp Cys Gln Val Asp Ile Ala Phe Pro Cys Ala Thr Gln Asn Glu Ile
              395           400           405


GAT GAG CAC GAC GCC GAG CTG CTG ATC AAG CAC GGC TGC CAG TAC GTG
1301
Asp Glu His Asp Ala Glu Leu Leu Ile Lys His Gly Cys Gln Tyr Val
              410           415           420
```

```
GTG GAG GGC GCC AAC ATG CCC TCC ACC AAC GAG GCC ATC CAC AAG TAC
1349
Val Glu Gly Ala Asn Met Pro Ser Thr Asn Glu Ala Ile His Lys Tyr
    425             430             435

AAC AAG GCC GGC ATC ATC TAC TGC CCC GGC AAG GCG GCC AAC GCC GGC
1397
Asn Lys Ala Gly Ile Ile Tyr Cys Pro Gly Lys Ala Ala Asn Ala Gly
440             445             450             455

GGC GTG GCG GTC AGC GGC CTG GAG ATG ACC CAG AAC CGC ATG AGC CTG
1445
Gly Val Ala Val Ser Gly Leu Glu Met Thr Gln Asn Arg Met Ser Leu
            460             465             470

AAC TGG ACT CGC GAG GAG GTT CGC GAC AAG CTG GAG CGC ATC ATG AAG
1493
Asn Trp Thr Arg Glu Glu Val Arg Asp Lys Leu Glu Arg Ile Met Lys
            475             480             485

GAC ATC TAC GAC TCC GCC ATG GGG CCG TCC CGC AGA TAC AAT GTT GAC
1541
Asp Ile Tyr Asp Ser Ala Met Gly Pro Ser Arg Arg Tyr Asn Val Asp
        490             495             500

CTG GCT GCG GGC GCC AAC ATC GCG GGC TTC ACC AAG GTG GCT GAT GCC
1589
Leu Ala Ala Gly Ala Asn Ile Ala Gly Phe Thr Lys Val Ala Asp Ala
    505             510             515

GTC AAG GCC CAG GGC GCT GTT TAAGCTGCCC AGGCCCAAGC CACGGCTCAC
1640
Val Lys Ala Gln Gly Ala Val
520             525

CGGCAATCCA ACCCAACCAA CTCAACGGCC AGGACCTTTT CGGAAGCGGC GCCTTTTTCC
1700

CAGCCAGGGC CCTCACCTGC CCTTTCATAA CCCTGCTATT GCCGCCGTGC CCCTGCAATT
1760

CCACCCCAAG AAGAACTAGC GGCACTTGAC TGCATCAGGA CGGCTATTTT TTTCGCGACG
1820

CGCGCTCACC CCGAGAGCCT CTCTCCCCCG AGCCCTAAGC GCTGACGTCC GCCCGACTTT
1880

GCCTCGCACA TCGCTCGGTT TTGACCCCCT CCAGTCTACC CACCCTGTTG TGAAGCCTAC
1940

CAGCTCAATT GCCTTTTAGT GTATGTGCGC CCCCTCCTGC CCCCGAATTT TCCTGCCATG
2000

AGACGTGCGG TTCCTAGCCT GGTGACCCCA AGTAGCAGTT AGTGTGCGTG CCTTGCCCTG
2060

CGCTGCCCGG GATGCGATAC TGTGACCTGA GAGTGCTTGT GTAAACACGA CGAGTCAAAA
2120

AAAAAAAAAA AAAAAAAAAA
2140
```

(2) INFORMATION FOR SEQ ID NO:2:

    (i) SEQUENCE CHARACTERISTICS:

(A) LENGTH: 526 amino acids
(B) TYPE: amino acid
(D) TOPOLOGY: linear

(ii) MOLECULE TYPE: protein
(xi) SEQUENCE DESCRIPTION: SEQ ID NO:2:

```
Met Gln Thr Ala Leu Val Ala Lys Pro Ile Val Ala Ala Pro Leu Ala
 1               5               10                  15

Ala Arg Pro Arg Cys Leu Ala Pro Trp Pro Cys Ala Trp Val Arg Ser
            20              25                  30

Ala Lys Arg Asp Val Arg Ala Lys Ala Val Ser Leu Glu Glu Gln Ile
        35              40                  45

Ser Ala Met Asp Ala Thr Thr Gly Asp Phe Thr Ala Leu Gln Lys Ala
    50              55                  60

Val Lys Gln Met Ala Thr Lys Ala Gly Thr Glu Gly Leu Val His Gly
65              70                  75                      80

Ile Lys Asn Pro Asp Val Arg Gln Leu Leu Thr Glu Ile Phe Met Lys
            85                  90                      95

Asp Pro Glu Gln Gln Glu Phe Met Gln Ala Val Arg Glu Val Ala Val
            100                 105                 110

Ser Leu Gln Pro Val Phe Glu Lys Arg Pro Glu Leu Leu Pro Ile Phe
        115                 120                 125

Lys Gln Ile Val Glu Pro Glu Arg Val Ile Thr Phe Arg Val Ser Trp
    130                 135                 140

Leu Asp Asp Ala Gly Asn Leu Gln Val Asn Arg Gly Phe Arg Val Gln
145                 150                 155                 160

Tyr Ser Ser Ala Ile Gly Pro Tyr Lys Gly Gly Leu Arg Phe His Pro
            165                 170                 175

Ser Val Asn Leu Ser Ile Met Lys Phe Leu Ala Phe Glu Gln Ile Phe
            180                 185                 190

Lys Asn Ser Leu Thr Thr Leu Pro Met Gly Gly Gly Lys Gly Gly Ser
        195                 200                 205

Asp Phe Asp Pro Lys Gly Lys Ser Asp Ala Glu Val Met Arg Phe Cys
    210                 215                 220

Gln Ser Phe Met Thr Glu Leu Gln Arg His Ile Ser Tyr Val Gln Asp
225                 230                 235                 240

Val Pro Ala Gly Asp Ile Gly Val Gly Ala Arg Glu Ile Gly Tyr Leu
            245                 250                 255

Phe Gly Gln Tyr Lys Arg Ile Thr Lys Asn Tyr Thr Gly Val Leu Thr
        260                 265                 270

Pro Lys Gly Gln Glu Tyr Gly Gly Ser Glu Ile Arg Pro Glu Ala Thr
    275                 280                 285

Gly Tyr Gly Ala Val Leu Phe Val Glu Asn Val Leu Lys Asp Lys Gly
    290                 295                 300

Glu Ser Leu Lys Gly Lys Arg Cys Leu Val Ser Gly Ala Gly Asn Val
305                 310                 315                 320
```

```
Ala Gln Tyr Cys Ala Glu Leu Leu Leu Glu Lys Gly Ala Ile Val Leu
                325             330                     335

Ser Leu Ser Asp Ser Gln Gly Tyr Val Tyr Glu Pro Asn Gly Phe Thr
                340             345                 350

Arg Glu Gln Leu Gln Ala Val Gln Asp Met Lys Lys Lys Asn Asn Ser
            355             360             365

Ala Arg Ile Ser Glu Tyr Lys Ser Asp Thr Ala Val Tyr Val Gly Asp
    370             375             380

Arg Arg Lys Pro Trp Glu Leu Asp Cys Gln Val Asp Ile Ala Phe Pro
385             390             395                     400

Cys Ala Thr Gln Asn Glu Ile Asp Glu His Asp Ala Glu Leu Leu Ile
            405             410             415

Lys His Gly Cys Gln Tyr Val Val Glu Gly Ala Asn Met Pro Ser Thr
            420             425             430

Asn Glu Ala Ile His Lys Tyr Asn Lys Ala Gly Ile Ile Tyr Cys Pro
        435             440             445

Gly Lys Ala Ala Asn Ala Gly Gly Val Ala Val Ser Gly Leu Glu Met
    450             455             460

Thr Gln Asn Arg Met Ser Leu Asn Trp Thr Arg Glu Glu Val Arg Asp
465             470             475                     480

Lys Leu Glu Arg Ile Met Lys Asp Ile Tyr Asp Ser Ala Met Gly Pro
            485             490                 495

Ser Arg Arg Tyr Asn Val Asp Leu Ala Ala Gly Ala Asn Ile Ala Gly
            500             505                 510

Phe Thr Lys Val Ala Asp Ala Val Lys Ala Gln Gly Ala Val
            515             520             525
```

(2) INFORMATION FOR SEQ ID NO:3:

    (i) SEQUENCE CHARACTERISTICS:

        (A) LENGTH: 2099 base pairs
        (B) TYPE: nucleic acid
        (C) STRANDEDNESS: double
        (D) TOPOLOGY: linear

    (ii) MOLECULE TYPE: cDNA
    (ix) FEATURE:

        (A) NAME/KEY: CDS
        (B) LOCATION: 33..1568

    (xi) SEQUENCE DESCRIPTION: SEQ ID NO:3:

```
CTCCTTTCTG CTCGCCCTCT CTCCGTCCCG CC ATG CAG ACC GCC CTC GTC GCC
53
                                      Met Gln Thr Ala Leu Val Ala
                                       1               5

AAG CCT ATC GTG GCC TGC GCG TGG GTC CGC TCC GCC AAG CGC GAT GTC
101
Lys Pro Ile Val Ala Cys Ala Trp Val Arg Ser Ala Lys Arg Asp Val
         10              15                20
```

CGC GCC AAG GCC GTC TCG CTG GAG GAG CAG ATC TCC GCG ATG GAC GCC
149
Arg Ala Lys Ala Val Ser Leu Glu Glu Gln Ile Ser Ala Met Asp Ala
    25                  30              35

ACC ACC GGC GAC TTC ACG GCG CTG CAG AAG GCG GTG AAG CAG ATG GCC
197
Thr Thr Gly Asp Phe Thr Ala Leu Gln Lys Ala Val Lys Gln Met Ala
40             45            50            55

ACC AAG GCG GGC ACT GAG GGC CTG GTG CAC GGC ATC AAG AAC CCC GAC
245
Thr Lys Ala Gly Thr Glu Gly Leu Val His Gly Ile Lys Asn Pro Asp
           60            65            70

GTG CGC CAG CTG CTG ACC GAG ATC TTC ATG AAG GAC CCG GAG CAG CAG
293
Val Arg Gln Leu Leu Thr Glu Ile Phe Met Lys Asp Pro Glu Gln Gln
           75            80            85

GAG TTC ATG CAG GCG GTG CGC GAG GTG GCC GTC TCC CTG CAG CCC GTG
341
Glu Phe Met Gln Ala Val Arg Glu Val Ala Val Ser Leu Gln Pro Val
         90            95          100

TTC GAG AAG CGC CCC GAG CTG CTG CCC ATC TTC AAG CAG ATC GTT GAG
389
Phe Glu Lys Arg Pro Glu Leu Leu Pro Ile Phe Lys Gln Ile Val Glu
    105           110          115

CCT GAG CGC GTG ATC ACC TTC CGC GTG TCC TGG CTG GAC GAC GCC GGC
437
Pro Glu Arg Val Ile Thr Phe Arg Val Ser Trp Leu Asp Asp Ala Gly
120          125          130          135

AAC CTG CAG GTC AAC CGC GGC TTC CGC GTG CAG TAC TCG TCC GCC ATC
485
Asn Leu Gln Val Asn Arg Gly Phe Arg Val Gln Tyr Ser Ser Ala Ile
           140           145           150

GGC CCC TAC AAG GGC GGC CTG CGC TTC CAC CCC TCC GTG AAC CTG TCC
533
Gly Pro Tyr Lys Gly Gly Leu Arg Phe His Pro Ser Val Asn Leu Ser
        155           160          165

ATC ATG AAG TTC CTT GCC TTT GAG CAG ATC TTC AAG AAC AGC CTG ACC
581
Ile Met Lys Phe Leu Ala Phe Glu Gln Ile Phe Lys Asn Ser Leu Thr
        170          175          180

ACC CTG CCC ATG GGC GGC GGC AAG GGC GGC TCC GAC TTC GAC CCC AAG
629
Thr Leu Pro Met Gly Gly Gly Lys Gly Gly Ser Asp Phe Asp Pro Lys
        185          190          195

GGC AAG AGC GAC GCG GAG GTG ATG CGC TTC TGC CAG TCC TTC ATG ACC
677
Gly Lys Ser Asp Ala Glu Val Met Arg Phe Cys Gln Ser Phe Met Thr
200          205          210          215

GAG CTG CAG CGC CAC ATC AGC TAC GTG CAG GAC GTG CCC GCC GGC GAC
725
Glu Leu Gln Arg His Ile Ser Tyr Val Gln Asp Val Pro Ala Gly Asp
        220          225          230

ATC GGC GTG GGC GCG CGC GAG ATT ｜C TAC CTT TTC GGC CAG TAC AAG
773

EP 0 859 849 B1

```
                Ile Gly Val Gly Ala Arg Glu Ile Gly Tyr Leu Phe Gly Gln Tyr Lys
                            235             240             245

                CGC ATC ACC AAG AAC TAC ACC GGC GTG CTG ACC CCG AAG GGC CAG GAG
                821
                Arg Ile Thr Lys Asn Tyr Thr Gly Val Leu Thr Pro Lys Gly Gln Glu
                            250             255             260

                TAT GGC GGC TCC GAG ATC CGC CCC GAG GCC ACC GGC TAC GGC GCC GTG
                869
                Tyr Gly Gly Ser Glu Ile Arg Pro Glu Ala Thr Gly Tyr Gly Ala Val
                            265             270             275

                CTG TTT GTG GAG AAC GTG CTG AAG GAC AAG GGC GAG AGC CTC AAG GGC
                917
                Leu Phe Val Glu Asn Val Leu Lys Asp Lys Gly Glu Ser Leu Lys Gly
                280             285             290             295

                AAG CGC TGC CTG GTG TCT GGC GCG GGC AAC GTG GCC CAG TAC TGC GCG
                965
                Lys Arg Cys Leu Val Ser Gly Ala Gly Asn Val Ala Gln Tyr Cys Ala
                            300             305             310

                GAG CTG CTG CTG GAG AAG GGC GCC ATC GTG CTG TCG CTG TCC GAC TCC
                1013
                Glu Leu Leu Leu Glu Lys Gly Ala Ile Val Leu Ser Leu Ser Asp Ser
                            315             320             325

                CAG GGC TAC GTG TAC GAG CCC AAC GGC TTC ACG CGC GAG CAG CTG CAG
                1061
                Gln Gly Tyr Val Tyr Glu Pro Asn Gly Phe Thr Arg Glu Gln Leu Gln
                            330             335             340

                GCG GTG CAG GAC ATG AAG AAG AAG AAC AAC AGC GCC CGC ATC TCC GAG
                1109
                Ala Val Gln Asp Met Lys Lys Lys Asn Asn Ser Ala Arg Ile Ser Glu
                            345             350             355

                TAC AAG AGC GAC ACC GCC GTG TAT GTG GGC GAC CGC CGC AAG CCT TGG
                1157
                Tyr Lys Ser Asp Thr Ala Val Tyr Val Gly Asp Arg Arg Lys Pro Trp
                360             365             370             375

                GAG CTG GAC TGC CAG GTG GAC ATC GCC TTC CCC TGC GCC ACC CAG AAC
                1205
                Glu Leu Asp Cys Gln Val Asp Ile Ala Phe Pro Cys Ala Thr Gln Asn
                            380             385             390

                GAG ATC GAT GAG CAC GAC GCC GAG CTG CTG ATC AAG CAC GGC TGC CAG
                1253
                Glu Ile Asp Glu His Asp Ala Glu Leu Leu Ile Lys His Gly Cys Gln
                            395             400             405

                TAC GTG GTG GAG GGC GCC AAC ATG CCC TCC ACC AAC GAG GCC ATC CAC
                1301
                Tyr Val Val Glu Gly Ala Asn Met Pro Ser Thr Asn Glu Ala Ile His
                            410             415             420

                AAG TAC AAC AAG GCC GGC ATC ATC TAC TGC CCC GGC AAG GCG GCC AAC
                1349
                Lys Tyr Asn Lys Ala Gly Ile Ile Tyr Cys Pro Gly Lys Ala Ala Asn
                            425             430             435

                GCC GGC GGC GTG GCG GTC AGC GGC CTG GAG ATG ACC CAG AAC CGC ATG
                1397
                Ala Gly Gly Val Ala Val Ser Gly Leu Glu Met Thr Gln Asn Arg Met
                440             445             450             455
```

23

```
AGC CTG AAC TGG ACT CGC GAG GAG GTT CGC GAC AAG CTG GAG CGC ATC
1445
Ser Leu Asn Trp Thr Arg Glu Glu Val Arg Asp Lys Leu Glu Arg Ile
            460             465             470

ATG AAG GAC ATC TAC GAC TCC GCC ATG GGG CCG TCC CGC AGA TAC AAT
1493
Met Lys Asp Ile Tyr Asp Ser Ala Met Gly Pro Ser Arg Arg Tyr Asn
            475             480             485

GTT GAC CTG GCT GCG GGC GCC AAC ATC GCG GGC TTC ACC AAG GTG GCT
1541
Val Asp Leu Ala Ala Gly Ala Asn Ile Ala Gly Phe Thr Lys Val Ala
            490             495             500

GAT GCC GTC AAG GCC CAG GGC GCT GTT TAAGCTGCCC AGGCCCAAGC
1588
Asp Ala Val Lys Ala Gln Gly Ala Val
    505             510

CACGGCTCAC CGGCAATCCA ACCCAACCAA CTCAACGGCC AGGACCTTTT CGGAAGCGGC
1648

GCCTTTTTCC CAGCCAGGGC CCTCACCTGC CCTTTCATAA CCCTGCTATT GCCGCCGTGC
1708

CCCTGCAATT CCACCCCAAG AAGAACTAGC GGCACTTGAC TGCATCAGGA CGGCTATTTT
1768

TTTCGCGACG CGCGCTCACC CCGAGAGCCT CTCTCCCCCG AGCCCTAAGC GCTGACGTCC
1828

GCCCGACTTT GCCTCGCACA TCGCTCGGTT TTGACCCCCT CCAGTCTACC CACCCTGTTG
1888

TGAAGCCTAC CAGCTCAATT GCCTTTTAGT GTATGTGCGC CCCCTCCTGC CCCCGAATTT
1948

TCCTGCCATG AGACGTGCGG TTCCTAGCCT GGTGACCCCA AGTAGCAGTT AGTGTGCGTG
2008

CCTTGCCCTG CGCTGCCCGG GATGCGATAC TGTGACCTGA GAGTGCTTGT GTAAACACGA
2068

CGAGTCAAAA AAAAAAAAAA AAAAAAAAA A
2099
```

(2) INFORMATION FOR SEQ ID NO:4:

    (i) SEQUENCE CHARACTERISTICS:

        (A) LENGTH: 512 amino acids
        (B) TYPE: amino acid
        (D) TOPOLOGY: linear

    (ii) MOLECULE TYPE: protein
    (xi) SEQUENCE DESCRIPTION: SEQ ID NO:4:

Met Gln Thr Ala Leu Val Ala Lys Pro Ile Val Ala Cys Ala Trp Val
1               5               10                  15

Arg Ser Ala Lys Arg Asp Val Arg Ala Lys Ala Val Ser Leu Glu Glu
            20              25                  30

Gln Ile Ser Ala Met Asp Ala Thr Thr Gly Asp Phe Thr Ala Leu Gln
            35              40                  45

```
Lys Ala Val Lys Gln Met Ala Thr Lys Ala Gly Thr Glu Gly Leu Val
    50                  55                  60

His Gly Ile Lys Asn Pro Asp Val Arg Gln Leu Leu Thr Glu Ile Phe
65                  70                  75                  80

Met Lys Asp Pro Glu Gln Gln Glu Phe Met Gln Ala Val Arg Glu Val
                85                  90                  95

Ala Val Ser Leu Gln Pro Val Phe Glu Lys Arg Pro Glu Leu Leu Pro
            100                 105                 110

Ile Phe Lys Gln Ile Val Glu Pro Glu Arg Val Ile Thr Phe Arg Val
        115                 120                 125

Ser Trp Leu Asp Asp Ala Gly Asn Leu Gln Val Asn Arg Gly Phe Arg
    130                 135                 140

Val Gln Tyr Ser Ser Ala Ile Gly Pro Tyr Lys Gly Gly Leu Arg Phe
145                 150                 155                 160

His Pro Ser Val Asn Leu Ser Ile Met Lys Phe Leu Ala Phe Glu Gln
                165                 170                 175

Ile Phe Lys Asn Ser Leu Thr Thr Leu Pro Met Gly Gly Gly Lys Gly
            180                 185                 190

Gly Ser Asp Phe Asp Pro Lys Gly Lys Ser Asp Ala Glu Val Met Arg
            195                 200                 205

Phe Cys Gln Ser Phe Met Thr Glu Leu Gln Arg His Ile Ser Tyr Val
    210                 215                 220

Gln Asp Val Pro Ala Gly Asp Ile Gly Val Gly Ala Arg Glu Ile Gly
225                 230                 235                 240

Tyr Leu Phe Gly Gln Tyr Lys Arg Ile Thr Lys Asn Tyr Thr Gly Val
            245                 250                 255

Leu Thr Pro Lys Gly Gln Glu Tyr Gly Gly Ser Glu Ile Arg Pro Glu
            260                 265                 270

Ala Thr Gly Tyr Gly Ala Val Leu Phe Val Glu Asn Val Leu Lys Asp
        275                 280                 285

Lys Gly Glu Ser Leu Lys Gly Lys Arg Cys Leu Val Ser Gly Ala Gly
    290                 295                 300

Asn Val Ala Gln Tyr Cys Ala Glu Leu Leu Leu Glu Lys Gly Ala Ile
305                 310                 315                 320

Val Leu Ser Leu Ser Asp Ser Gln Gly Tyr Val Tyr Glu Pro Asn Gly
                325                 330                 335

Phe Thr Arg Glu Gln Leu Gln Ala Val Gln Asp Met Lys Lys Lys Asn
        340                 345                 350

Asn Ser Ala Arg Ile Ser Glu Tyr Lys Ser Asp Thr Ala Val Tyr Val
        355                 360                 365

Gly Asp Arg Arg Lys Pro Trp Glu Leu Asp Cys Gln Val Asp Ile Ala
370                 375                 380

Phe Pro Cys Ala Thr Gln Asn Glu Ile Asp Glu His Asp Ala Glu Leu
385                 390                 395                 400
```

26

```
Leu Ile Lys His Gly Cys Gln Tyr Val Val Glu Gly Ala Asn Met Pro
            405         410                 415

Ser Thr Asn Glu Ala Ile His Lys Tyr Asn Lys Ala Gly Ile Ile Tyr
            420             425                 430

Cys Pro Gly Lys Ala Ala Asn Ala Gly Gly Val Ala Val Ser Gly Leu
            435             440                 445

Glu Met Thr Gln Asn Arg Met Ser Leu Asn Trp Thr Arg Glu Glu Val
    450             455             460

Arg Asp Lys Leu Glu Arg Ile Met Lys Asp Ile Tyr Asp Ser Ala Met
465             470             475                 480

Gly Pro Ser Arg Arg Tyr Asn Val Asp Leu Ala Ala Gly Ala Asn Ile
            485                 490                 495

Ala Gly Phe Thr Lys Val Ala Asp Ala Val Lys Ala Gln Gly Ala Val
            500             505                 510
```

(2) INFORMATION FOR SEQ ID NO:5:

   (i) SEQUENCE CHARACTERISTICS:

      (A) LENGTH: 20 amino acids
      (B) TYPE: amino acid
      (C) STRANDEDNESS: single
      (D) TOPOLOGY: linear

   (ii) MOLECULE TYPE: peptide
   (xi) SEQUENCE DESCRIPTION: SEQ ID NO:5:

```
Ala Val Ser Leu Glu Glu Gln Ile Ser Ala Met Asp Ala Thr Thr Gly
1               5                   10                  15

Asp Phe Thr Ala
            20
```

(2) INFORMATION FOR SEQ ID NO:6:

   (i) SEQUENCE CHARACTERISTICS:

      (A) LENGTH: 10 amino acids
      (B) TYPE: amino acid
      (C) STRANDEDNESS: single
      (D) TOPOLOGY: linear

   (ii) MOLECULE TYPE: peptide
   (xi) SEQUENCE DESCRIPTION: SEQ ID NO:6:

```
Asp Ala Thr Thr Gly Asp Phe Thr Ala Leu
1               5                   10
```

(2) INFORMATION FOR SEQ ID NO:7:

(i) SEQUENCE CHARACTERISTICS:

    (A) LENGTH: 1969 base pairs
    (B) TYPE: nucleic acid
    (C) STRANDEDNESS: double
    (D) TOPOLOGY: linear

(ii) MOLECULE TYPE: cDNA
(xi) SEQUENCE DESCRIPTION: SEQ ID NO:7:

```
CAGATCTCCG CGATGGACGC CACCACCGGC GACTTCACGG CGCTGCAGAA GGCGGTGAAG
60

CAGATGGCCA CCAAGGCGGG CACTGAGGGC CTGGTGCACG GCATCAAGAA CCCCGACGTG
120

CGCCAGCTGC TGACCGAGAT CTTCATGAAG GACCCGGAGC AGCAGGAGTT CATGCAGGCG
180

GTGCGCGAGG TGGCCGTCTC CCTGCAGCCC GTGTTCGAGA AGCGCCCCGA GCTGCTGCCC
240

ATCTTCAAGC AGATCGTTGA GCCTGAGCGC GTGATCACCT TCCGCGTGTC CTGGCTGGAC
300

GACGCCGGCA ACCTGCAGGT CAACCGCGGC TTCCGCGTGC AGTACTCGTC CGCCATCGGC
360

CCCTACAAGG GCGGCCTGCG CTTCCACCCC TCCGTGAACC TGTCCATCAT GAAGTTCCTT
420

GCCTTTGAGC AGATCTTCAA GAACAGCCTG ACCACCCTGC CCATGGGCGG CGGCAAGGGC
480

GGCTCCGACT TCGACCCCAA GGGCAAGAGC GACGCGGAGG TGATGCGCTT CTGCCAGTCC
540

TTCATGACCG AGCTGCAGCG CCACATCAGC TACGTGCAGG ACGTGCCCGC CGGCGACATC
600

GGCGTGGGCG CGCGCGAGAT TGGCTACCTT TTCGGCCAGT ACAAGCGCAT CACCAAGAAC
660

TACACCGGCG TGCTGACCCC GAAGGGCCAG GAGTATGGCG GCTCCGAGAT CCGCCCCGAG
720

GCCACCGGCT ACGGCGCCGT GCTGTTTGTG GAGAACGTGC TGAAGGACAA GGGCGAGAGC
780

CTCAAGGGCA AGCGCTGCCT GGTGTCTGGC GCGGGCAACG TGGCCCAGTA CTGCGCGGAG
840

CTGCTGCTGG AGAAGGGCGC CATCGTGCTG TCGCTGTCCG ACTCCCAGGG CTACGTGTAC
900

GAGCCCAACG GCTTCACGCG CGAGCAGCTG CAGGCGGTGC AGGACATGAA GAAGAAGAAC
960

AACAGCGCCC GCATCTCCGA GTACAAGAGC GACACCGCCG TGTATGTGGG CGACCGCCGC
1020

AAGCCTTGGG AGCTGGACTG CCAGGTGGAC ATCGCCTTCC CCTGCGCCAC CCAGAACGAG
1080

ATCGATGAGC ACGACGCCGA GCTGCTGATC AAGCACGGCT GCCAGTACGT GGTGGAGGGC
1140

GCCAACATGC CCTCCACCAA CGAGGCCATC CACAAGTACA ACAAGGCCGG CATCATCTAC
1200

TGCCCCGGCA AGGCGGCCAA CGCCGGCGGC GTGGCGGTCA GCGGCCTGGA GATGACCCAG
1260
```

29

```
AACCGCATGA GCCTGAACTG GACTCGCGAG GAGGTTCGCG ACAAGCTGGA GCGCATCATG
1320

AAGGACATCT ACGACTCCGC CATGGGGCCG TCCCGCAGAT ACAATGTTGA CCTGGCTGCG
1380

GGCGCCAACA TCGCGGGCTT CACCAAGGTG GCTGATGCCG TCAAGGCCCA GGGCGCTGTT
1440

TAAGCTGCCC AGGCCCAAGC CACGGCTCAC CGGCAATCCA ACCCAACCAA CTCAACGGCC
1500

AGGACCTTTT CGGAAGCGGC GCCTTTTTCC CAGCCAGGGC CCTCACCTGC CCTTTCATAA
1560

CCCTGCTATT GCCGCCGTGC CCCTGCAATT CCACCCCAAG AAGAACTAGC GGCACTTGAC
1620

TGCATCAGGA CGGCTATTTT TTTCGCGACG CGCGCTCACC CCGAGAGCCT CTCTCCCCCG
1680

AGCCCTAAGC GCTGACGTCC GCCCGACTTT GCCTCGCACA TCGCTCGGTT TTGACCCCCT
1740

CCAGTCTACC CACCCTGTTG TGAAGCCTAC CAGCTCAATT GCCTTTTAGT GTATGTGCGC
1800

CCCCTCCTGC CCCCGAATTT TCCTGCCATG AGACGTGCGG TTCCTAGCCT GGTGACCCCA
1860

AGTAGCAGTT AGTGTGCGTG CCTTGCCCTG CGCTGCCCGG GATGCGATAC TGTGACCTGA
1920

GAGTGCTTGT GTAAACACGA CGAGTCAAAA AAAAAAAAAA AAAAAAAA
1969
```

(2) INFORMATION FOR SEQ ID NO:8:

  (i) SEQUENCE CHARACTERISTICS:

    (A) LENGTH: 22 base pairs
    (B) TYPE: nucleic acid
    (C) STRANDEDNESS: double
    (D) TOPOLOGY: linear

  (ii) MOLECULE TYPE: cDNA
  (xi) SEQUENCE DESCRIPTION: SEQ ID NO:8:

```
                    CTCAAAGGCA AGGAACTTCA TG
                    22
```

(2) INFORMATION FOR SEQ ID NO:9:

  (i) SEQUENCE CHARACTERISTICS:

    (A) LENGTH: 50 base pairs
    (B) TYPE: nucleic acid
    (C) STRANDEDNESS: double
    (D) TOPOLOGY: linear

  (ii) MOLECULE TYPE: cDNA

(xi) SEQUENCE DESCRIPTION: SEQ ID NO:9:

```
GGGTCGACAT TCTAGACAGA ATTCGTGGAT CCTTTTTTTT TTTTTTTTTT
50
```

(2) INFORMATION FOR SEQ ID NO:10:

(i) SEQUENCE CHARACTERISTICS:

(A) LENGTH: 17 base pairs
(B) TYPE: nucleic acid
(C) STRANDEDNESS: double
(D) TOPOLOGY: linear

(ii) MOLECULE TYPE: cDNA
(xi) SEQUENCE DESCRIPTION: SEQ ID NO:10:

```
GGACGAGTAC TGCACGC
17
```

(2) INFORMATION FOR SEQ ID NO:11:

(i) SEQUENCE CHARACTERISTICS:

(A) LENGTH: 18 base pairs
(B) TYPE: nucleic acid
(C) STRANDEDNESS: double
(D) TOPOLOGY: linear

(ii) MOLECULE TYPE: CDNA
(xi) SEQUENCE DESCRIPTION: SEQ ID NO:11:

```
GATCTCGGTC AGCAGCTG
18
```

(2) INFORMATION FOR SEQ ID NO:12:

(i) SEQUENCE CHARACTERISTICS:

(A) LENGTH: 21 base pairs
(B) TYPE: nucleic acid
(C) STRANDEDNESS: double
(D) TOPOLOGY: linear

(ii) MOLECULE TYPE: CDNA
(xi) SEQUENCE DESCRIPTION: SEQ ID NO:12:

```
GGGTCGACAT TCTAGACAGA A
21
```

(2) INFORMATION FOR SEQ ID NO:13:

(i) SEQUENCE CHARACTERISTICS:

(A) LENGTH: 367 base pairs
(B) TYPE: nucleic acid
(C) STRANDEDNESS: double
(D) TOPOLOGY: linear

(ii) MOLECULE TYPE: CDNA
(xi) SEQUENCE DESCRIPTION: SEQ ID NO:13:

```
GGGTCGACAT TCTAGACAGA ATTCGTGGAT CCTTTTTTTT TTTTTTTTTT TTTTTTCTCC
60

TTTCTGCTCG CCCTCTCTCC GTCCCGCCAT GCAGACCGCC CTCGTCGCCA AGCCTATCGT
120

GGCCGCCCCG CTGGCGGCAC GCCCGCGCTG CCTCGCGCCG TGGCCGTGCG CGTGGGTCCG
180

CTCCGCCAAG CGCGATGTCC GCGCCAAGGC CGTCTCGCTG GAGGAGCAGA TCTCCGCGAT
240

GGACGCCACC ACCGGCGACT TCACGGCGCT GCAGAAGGCG GTGAAGCAGA TGGCCACCAA
300

GGCGGGCACT GAGGGCCTGG TGCACGGCAT CAAGAACCCC GACGTGCGCC AGCTGCTGAC
360

CGAGATC
367
```

(2) INFORMATION FOR SEQ ID NO:14:

(i) SEQUENCE CHARACTERISTICS:

(A) LENGTH: 325 base pairs
(B) TYPE: nucleic acid
(C) STRANDEDNESS: double
(D) TOPOLOGY: linear

(ii) MOLECULE TYPE: CDNA
(xi) SEQUENCE DESCRIPTION: SEQ ID NO:14:

```
GGGTCGACAT TCTAGACAGA ATTCGTGGAT CCTTTTTTTT TTTTTTTTTT TTTTTTCTCC
60

TTTCTGCTCG CCCTCTCTCC GTCCCGCCAT GCAGACCGCC CTCGTCGCCA AGCCTATCGT
120

GGCCTGCGCG TGGGTCCGCT CCGCCAAGCG CGATGTCCGC GCCAAGGCCG TCTCGCTGGA
180

GGAGCAGATC TCCGCGATGG ACGCCACCAC CGGCGACTTC ACGGCGCTGC AGAAGGCGGT
240

GAAGCAGATG GCCACCAAGG CGGGCACTGA GGGCCTGGTG CACGGCATCA AGAACCCCGA
300

CGTGCGCCAG CTGCTGACCG AGATC
325
```

(2) INFORMATION FOR SEQ ID NO:15:

    (i) SEQUENCE CHARACTERISTICS:

        (A) LENGTH: 18 base pairs
        (B) TYPE: nucleic acid
        (C) STRANDEDNESS: double
        (D) TOPOLOGY: linear

    (ii) MOLECULE TYPE: CDNA
    (xi) SEQUENCE DESCRIPTION: SEQ ID NO:15:

```
                              CTTTCTGCTC GCCCTCTC
                              18
```

(2) INFORMATION FOR SEQ ID NO:16:

    (i) SEQUENCE CHARACTERISTICS:

        (A) LENGTH: 308 base pairs
        (B) TYPE: nucleic acid
        (C) STRANDEDNESS: double
        (D) TOPOLOGY: linear

    (ii) MOLECULE TYPE: CDNA
    (xi) SEQUENCE DESCRIPTION: SEQ ID NO:16:

```
CTTTCTGCTC GCCCTCTCTC CGTCCCGCCA TGCAGACCGC CCTCGTCGCC AAGCCTATCG
60

TGGCCGCCCC GCTGGCGGCA CGCCCGCGCT GCCTCGCGCC GTGGCCGTGC GCGTGGGTCC
120

GCTCCGCCAA GCGCGATGTC CGCGCCAAGG CCGTCTCGCT GGAGGAGCAG ATCTCCGCGA
180

TGGACGCCAC CACCGGCGAC TTCACGGCGC TGCAGAAGGC GGTGAAGCAG ATGGCCACCA
240

AGGCGGGCAC TGAGGGCCTG GTGCACGGCA TCAAGAACCC CGACGTGCGC CAGCTGCTGA
300

CCGAGATC
308
```

(2) INFORMATION FOR SEQ ID NO:17:

    (i) SEQUENCE CHARACTERISTICS:

        (A) LENGTH: 266 base pairs
        (B) TYPE: nucleic acid
        (C) STRANDEDNESS: double
        (D) TOPOLOGY: linear

    (ii) MOLECULE TYPE: CDNA
    (xi) SEQUENCE DESCRIPTION: SEQ ID NO:17:

```
CTTTCTGCTC GCCCTCTCTC CGTCCCGCCA TGCAGACCGC CCTCGTCGCC AAGCCTATCG
60

TGGCCTGCGC GTGGGTCCGC TCCGCCAAGC GCGATGTCCG CGCCAAGGCC GTCTCGCTGG
120

AGGAGCAGAT CTCCGCGATG GACGCCACCA CCGGCGACTT CACGGCGCTG CAGAAGGCGG
180

TGAAGCAGAT GGCCACCAAG GCGGGCACTG AGGGCCTGGT GCACGGCATC AAGAACCCCG
240

ACGTGCGCCA GCTGCTGACC GAGATC
266
```

(2) INFORMATION FOR SEQ ID NO:18:

   (i) SEQUENCE CHARACTERISTICS:

      (A) LENGTH: 2137 base pairs
      (B) TYPE: nucleic acid
      (C) STRANDEDNESS: double
      (D) TOPOLOGY: linear

   (ii) MOLECULE TYPE: CDNA
   (xi) SEQUENCE DESCRIPTION: SEQ ID NO:18:

CTTTCTGCTC GCCCTCTCTC CGTCCCGCCA TGCAGACCGC CCTCGTCGCC AAGCCTATCG
60

TGGCCGCCCC GCTGGCGGCA CGCCCGCGCT GCCTCGCGCC GTGGCCGTGC GCGTGGGTCC
120

GCTCCGCCAA GCGCGATGTC CGCGCCAAGG CCGTCTCGCT GGAGGAGCAG ATCTCCGCGA
180

TGGACGCCAC CACCGGCGAC TTCACGGCGC TGCAGAAGGC GGTGAAGCAG ATGGCCACCA
240

AGGCGGGCAC TGAGGGCCTG GTGCACGGCA TCAAGAACCC CGACGTGCGC CAGCTGCTGA
300

CCGAGATCTT CATGAAGGAC CCGGAGCAGC AGGAGTTCAT GCAGGCGGTG CGCGAGGTGG
360

CCGTCTCCCT GCAGCCCGTG TTCGAGAAGC GCCCCGAGCT GCTGCCCATC TTCAAGCAGA
420

TCGTTGAGCC TGAGCGCGTG ATCACCTTCC GCGTGTCCTG GCTGGACGAC GCCGGCAACC
480

TGCAGGTCAA CCGCGGCTTC CGCGTGCAGT ACTCGTCCGC CATCGGCCCC TACAAGGGCG
540

GCCTGCGCTT CCACCCCTCC GTGAACCTGT CCATCATGAA GTTCCTTGCC TTTGAGCAGA
600

TCTTCAAGAA CAGCCTGACC ACCCTGCCCA TGGGCGGCGG CAAGGGCGGC TCCGACTTCG
660

ACCCCAAGGG CAAGAGCGAC GCGGAGGTGA TGCGCTTCTG CCAGTCCTTC ATGACCGAGC
720

TGCAGCGCCA CATCAGCTAC GTGCAGGACG TGCCCGCCGG CGACATCGGC GTGGGCGCGC
780

GCGAGATTGG CTACCTTTTC GGCCAGTACA AGCGCATCAC CAAGAACTAC ACCGGCGTGC
840

TGACCCCGAA GGGCCAGGAG TATGGCGGCT CCGAGATCCG CCCCGAGGCC ACCGGCTACG
900

GCGCCGTGCT GTTTGTGGAG AACGTGCTGA AGGACAAGGG CGAGAGCCTC AAGGGCAAGC
960

GCTGCCTGGT GTCTGGCGCG GGCAACGTGG CCCAGTACTG CGCGGAGCTG CTGCTGGAGA
1020

AGGGCGCCAT CGTGCTGTCG CTGTCCGACT CCCAGGGCTA CGTGTACGAG CCCAACGGCT
1080

TCACGCGCGA GCAGCTGCAG GCGGTGCAGG ACATGAAGAA GAAGAACAAC AGCGCCCGCA
1140

TCTCCGAGTA CAAGAGCGAC ACCGCCGTGT ATGTGGGCGA CCGCCGCAAG CCTTGGGAGC
1200

TGGACTGCCA GGTGGACATC GCCTTCCCCT GCGCCACCCA GAACGAGATC GATGAGCACG
1260

ACGCCGAGCT GCTGATCAAG CACGGCTGCC AGTACGTGGT GGAGGGCGCC AACATGCCCT
1320

```
CCACCAACGA GGCCATCCAC AAGTACAACA AGGCCGGCAT CATCTACTGC CCCGGCAAGG
1380

CGGCCAACGC CGGCGGCGTG GCGGTCAGCG GCCTGGAGAT GACCCAGAAC CGCATGAGCC
1440

TGAACTGGAC TCGCGAGGAG GTTCGCGACA AGCTGGAGCG CATCATGAAG GACATCTACG
1500

ACTCCGCCAT GGGGCCGTCC CGCAGATACA ATGTTGACCT GGCTGCGGGC GCCAACATCG
1560

CGGGCTTCAC CAAGGTGGCT GATGCCGTCA AGGCCCAGGG CGCTGTTTAA GCTGCCCAGG
1620

CCCAAGCCAC GGCTCACCGG CAATCCAACC CAACCAACTC AACGGCCAGG ACCTTTTCGG
1680

AAGCGGCGCC TTTTTCCCAG CCAGGGCCCT CACCTGCCCT TTCATAACCC TGCTATTGCC
1740

GCCGTGCCCC TGCAATTCCA CCCCAAGAAG AACTAGCGGC ACTTGACTGC ATCAGGACGG
1800

CTATTTTTTT CGCGACGCGC GCTCACCCCG AGAGCCTCTC TCCCCCGAGC CCTAAGCGCT
1860

GACGTCCGCC CGACTTTGCC TCGCACATCG CTCGGTTTTG ACCCCCTCCA GTCTACCCAC
1920

CCTGTTGTGA AGCCTACCAG CTCAATTGCC TTTTAGTGTA TGTGCGCCCC CTCCTGCCCC
1980

CGAATTTTCC TGCCATGAGA CGTGCGGTTC CTAGCCTGGT GACCCCAAGT AGCAGTTAGT
2040

GTGCGTGCCT TGCCCTGCGC TGCCCGGGAT GCGATACTGT GACCTGAGAG TGCTTGTGTA
2100

AACACGACGA GTCAAAAAAA AAAAAAAAAA AAAAAAA
2137
```

(2) INFORMATION FOR SEQ ID NO:19:

    (i) SEQUENCE CHARACTERISTICS:

        (A) LENGTH: 2096 base pairs
        (B) TYPE: nucleic acid
        (C) STRANDEDNESS: double
        (D) TOPOLOGY: linear

    (ii) MOLECULE TYPE: CDNA
    (xi) SEQUENCE DESCRIPTION: SEQ ID NO:19:

```
CTTTCTGCTC GCCCTCTCTC CGTCCCGCCA TGCAGACCGC CCTCGTCGCC AAGCCTATCG
60

TGGCCTGCGC GTGGGTCCGC TCCGCCAAGC GCGATGTCCG CGCCAAGGCC GTCTCGCTGG
120

AGGAGCAGAT CTCCGCGATG GACGCCACCA CCGGCGACTT CACGGCGCTG CAGAAGGCGG
180

TGAAGCAGAT GGCCACCAAG GCGGGCACTG AGGGCCTGGT GCACGGCATC AAGAACCCCG
240
```

ACGTGCGCCA GCTGCTGACC GAGATCTTCA TGAAGGACCC GGAGCAGCAG GAGTTCATGC
300

AGGCGGTGCG CGAGGTGGCC GTCTCCCTGC AGCCCGTGTT CGAGAAGCGC CCCGAGCTGC
360

TGCCCATCTT CAAGCAGATC GTTGAGCCTG AGCGCGTGAT CACCTTCCGC GTGTCCTGGC
420

TGGACGACGC CGGCAACCTG CAGGTCAACC GCGGCTTCCG CGTGCAGTAC TCGTCCGCCA
480

TCGGCCCCTA CAAGGGCGGC CTGCGCTTCC ACCCCTCCGT GAACCTGTCC ATCATGAAGT
540

TCCTTGCCTT TGAGCAGATC TTCAAGAACA GCCTGACCAC CCTGCCCATG GGCGGCGGCA
600

AGGGCGGCTC CGACTTCGAC CCCAAGGGCA AGAGCGACGC GGAGGTGATG CGCTTCTGCC
660

AGTCCTTCAT GACCGAGCTG CAGCGCCACA TCAGCTACGT GCAGGACGTG CCCGCCGGCG
720

ACATCGGCGT GGGCGCGCGC GAGATTGGCT ACCTTTTCGG CCAGTACAAG CGCATCACCA
780

AGAACTACAC CGGCGTGCTG ACCCCGAAGG GCCAGGAGTA TGGCGGCTCC GAGATCCGCC
840

CCGAGGCCAC CGGCTACGGC GCCGTGCTGT TTGTGGAGAA CGTGCTGAAG GACAAGGGCG
900

AGAGCCTCAA GGGCAAGCGC TGCCTGGTGT CTGGCGCGGG CAACGTGGCC CAGTACTGCG
960

CGGAGCTGCT GCTGGAGAAG GGCGCCATCG TGCTGTCGCT GTCCGACTCC CAGGGCTACG
1020

TGTACGAGCC CAACGGCTTC ACGCGCGAGC AGCTGCAGGC GGTGCAGGAC ATGAAGAAGA
1080

AGAACAACAG CGCCCGCATC TCCGAGTACA AGAGCGACAC CGCCGTGTAT GTGGGCGACC
1140

GCCGCAAGCC TTGGGAGCTG GACTGCCAGG TGGACATCGC CTTCCCCTGC GCCACCCAGA
1200

ACGAGATCGA TGAGCACGAC GCCGAGCTGC TGATCAAGCA CGGCTGCCAG TACGTGGTGG
1260

AGGGCGCCAA CATGCCCTCC ACCAACGAGG CCATCCACAA GTACAACAAG GCCGGCATCA
1320

TCTACTGCCC CGGCAAGGCG GCCAACGCCG GCGGCGTGGC GGTCAGCGGC CTGGAGATGA
1380

CCCAGAACCG CATGAGCCTG AACTGGACTC GCGAGGAGGT TCGCGACAAG CTGGAGCGCA
1440

TCATGAAGGA CATCTACGAC TCCGCCATGG GGCCGTCCCG CAGATACAAT GTTGACCTGG
1500

CTGCGGGCGC CAACATCGCG GGCTTCACCA AGGTGGCTGA TGCCGTCAAG GCCCAGGGCG
1560

38

```
CTGTTTAAGC TGCCCAGGCC CAAGCCACGG CTCACCGGCA ATCCAACCCA ACCAACTCAA
1620

CGGCCAGGAC CTTTTCGGAA GCGGCGCCTT TTTCCCAGCC AGGGCCCTCA CCTGCCCTTT
1680

CATAACCCTG CTATTGCCGC CGTGCCCCTG CAATTCCACC CCAAGAAGAA CTAGCGGCAC
1740

TTGACTGCAT CAGGACGGCT ATTTTTTTCG CGACGCGCGC TCACCCCGAG AGCCTCTCTC
1800

CCCCGAGCCC TAAGCGCTGA CGTCCGCCCG ACTTTGCCTC GCACATCGCT CGGTTTTGAC
1860

CCCCTCCAGT CTACCCACCC TGTTGTGAAG CCTACCAGCT CAATTGCCTT TTAGTGTATG
1920

TGCGCCCCCT CCTGCCCCCG AATTTTCCTG CCATGAGACG TGCGGTTCCT AGCCTGGTGA
1980

CCCCAAGTAG CAGTTAGTGT GCGTGCCTTG CCCTGCGCTG CCCGGGATGC GATACTGTGA
2040

CCTGAGAGTG CTTGTGTAAA CACGACGAGT CAAAAAAAAA AAAAAAAAAA AAAAAA
2096
```

(2) INFORMATION FOR SEQ ID NO:20:

(i) SEQUENCE CHARACTERISTICS:

(A) LENGTH: 25 base pairs
(B) TYPE: nucleic acid
(C) STRANDEDNESS: double
(D) TOPOLOGY: linear

(ii) MOLECULE TYPE: CDNA
(xi) SEQUENCE DESCRIPTION: SEQ ID NO:20:

```
CATATGGCCG TCTCGCTGGG AGGAG
25
```

(2) INFORMATION FOR SEQ ID NO:21:

(i) SEQUENCE CHARACTERISTICS:

(A) LENGTH: 19 base pairs
(B) TYPE: nucleic acid
(C) STRANDEDNESS: double
(D) TOPOLOGY: linear

(ii) MOLECULE TYPE: CDNA
(xi) SEQUENCE DESCRIPTION: SEQ ID NO:21:

```
GTTGGATTGC CGGTGAGCC
19
```

(2) INFORMATION FOR SEQ ID NO:22:

    (i) SEQUENCE CHARACTERISTICS:

        (A) LENGTH: 21 base pairs
        (B) TYPE: nucleic acid
        (C) STRANDEDNESS: double
        (D) TOPOLOGY: linear

    (ii) MOLECULE TYPE: CDNA
    (xi) SEQUENCE DESCRIPTION: SEQ ID NO:22:

```
CATATGGACG CCACCACCGG C
21
```

(2) INFORMATION FOR SEQ ID NO:23:

    (i) SEQUENCE CHARACTERISTICS:

        (A) LENGTH: 1506 base pairs
        (B) TYPE: nucleic acid
        (C) STRANDEDNESS: double
        (D) TOPOLOGY: linear

    (ii) MOLECULE TYPE: CDNA
    (ix) FEATURE:

        (A) NAME/KEY: CDS
        (B) LOCATION: 4..1464

    (xi) SEQUENCE DESCRIPTION: SEQ ID NO:24:

```
CAT ATG GCC GTC TCG CTG GAG GAG CAG ATC TCC GCG ATG GAC GCC ACC
48
    Met Ala Val Ser Leu Glu Glu Gln Ile Ser Ala Met Asp Ala Thr
            515             520             525

ACC GGC GAC TTC ACG GCG CTG CAG AAG GCG GTG AAG CAG ATG GCC ACC
96
Thr Gly Asp Phe Thr Ala Leu Gln Lys Ala Val Lys Gln Met Ala Thr
        530             535             540

AAG GCG GGC ACT GAG GGC CTG GTG CAC GGC ATC AAG AAC CCC GAC GTG
144
Lys Ala Gly Thr Glu Gly Leu Val His Gly Ile Lys Asn Pro Asp Val
    545             550             555

CGC CAG CTG CTG ACC GAG ATC TTC ATG AAG GAC CCG GAG CAG CAG GAG
192
Arg Gln Leu Leu Thr Glu Ile Phe Met Lys Asp Pro Glu Gln Gln Glu
560             565             570             575

TTC ATG CAG GCG GTG CGC GAG GTG GCC GTC TCC CTG CAG CCC GTG TTC
240
Phe Met Gln Ala Val Arg Glu Val Ala Val Ser Leu Gln Pro Val Phe
            580             585             590

GAG AAG CGC CCC GAG CTG CTG CCC ATC TTC AAG CAG ATC GTT GAG CCT
288
Glu Lys Arg Pro Glu Leu Leu Pro Ile Phe Lys Gln Ile Val Glu Pro
            595             600             605

GAG CGC GTG ATC ACC TTC CGC GTG TCC TGG CTG GAC GAC GCC GGC AAC
336
Glu Arg Val Ile Thr Phe Arg Val Ser Trp Leu Asp Asp Ala Gly Asn
        610             615             620

CTG CAG GTC AAC CGC GGC TTC CGC GTG CAG TAC TCG TCC GCC ATC GGC
384
Leu Gln Val Asn Arg Gly Phe Arg Val Gln Tyr Ser Ser Ala Ile Gly
    625             630             635
```

```
CCC TAC AAG GGC GGC CTG CGC TTC CAC CCC TCC GTG AAC CTG TCC ATC
432
Pro Tyr Lys Gly Gly Leu Arg Phe His Pro Ser Val Asn Leu Ser Ile
640             645         650             655

ATG AAG TTC CTT GCC TTT GAG CAG ATC TTC AAG AAC AGC CTG ACC ACC
480
Met Lys Phe Leu Ala Phe Glu Gln Ile Phe Lys Asn Ser Leu Thr Thr
        660         665             670

CTG CCC ATG GGC GGC GGC AAG GGC GGC TCC GAC TTC GAC CCC AAG GGC
528
Leu Pro Met Gly Gly Gly Lys Gly Gly Ser Asp Phe Asp Pro Lys Gly
        675         680             685

AAG AGC GAC GCG GAG GTG ATG CGC TTC TGC CAG TCC TTC ATG ACC GAG
576
Lys Ser Asp Ala Glu Val Met Arg Phe Cys Gln Ser Phe Met Thr Glu
        690         695             700

CTG CAG CGC CAC ATC AGC TAC GTG CAG GAC GTG CCC GCC GGC GAC ATC
624
Leu Gln Arg His Ile Ser Tyr Val Gln Asp Val Pro Ala Gly Asp Ile
        705         710         715

GGC GTG GGC GCG CGC GAG ATT GGC TAC CTT TTC GGC CAG TAC AAG CGC
672
Gly Val Gly Ala Arg Glu Ile Gly Tyr Leu Phe Gly Gln Tyr Lys Arg
720         725             730             735

ATC ACC AAG AAC TAC ACC GGC GTG CTG ACC CCG AAG GGC CAG GAG TAT
720
Ile Thr Lys Asn Tyr Thr Gly Val Leu Thr Pro Lys Gly Gln Glu Tyr
            740         745             750

GGC GGC TCC GAG ATC CGC CCC GAG GCC ACC GGC TAC GGC GCC GTG CTG
768
Gly Gly Ser Glu Ile Arg Pro Glu Ala Thr Gly Tyr Gly Ala Val Leu
            755         760             765

TTT GTG GAG AAC GTG CTG AAG GAC AAG GGC GAG AGC CTC AAG GGC AAG
816
Phe Val Glu Asn Val Leu Lys Asp Lys Gly Glu Ser Leu Lys Gly Lys
        770         775             780

CGC TGC CTG GTG TCT GGC GCG GGC AAC GTG GCC CAG TAC TGC GCG GAG
864
Arg Cys Leu Val Ser Gly Ala Gly Asn Val Ala Gln Tyr Cys Ala Glu
        785         790             795

CTG CTG CTG GAG AAG GGC GCC ATC GTG CTG TCG CTG TCC GAC TCC CAG
912
Leu Leu Leu Glu Lys Gly Ala Ile Val Leu Ser Leu Ser Asp Ser Gln
800             805         810             815

GGC TAC GTG TAC GAG CCC AAC GGC TTC ACG CGC GAG CAG CTG CAG GCG
960
Gly Tyr Val Tyr Glu Pro Asn Gly Phe Thr Arg Glu Gln Leu Gln Ala
            820         825             830

GTG CAG GAC ATG AAG AAG AAG AAC AAC AGC GCC CGC ATC TCC GAG TAC
1008
Val Gln Asp Met Lys Lys Lys Asn Asn Ser Ala Arg Ile Ser Glu Tyr
            835         840             845

AAG AGC GAC ACC GCC GTG TAT GTG GGC GAC CGC CGC AAG CCT TGG GAG
1056
```

42

```
Lys Ser Asp Thr Ala Val Tyr Val Gly Asp Arg Arg Lys Pro Trp Glu
        850             855             860

CTG GAC TGC CAG GTG GAC ATC GCC TTC CCC TGC GCC ACC CAG AAC GAG
1104
Leu Asp Cys Gln Val Asp Ile Ala Phe Pro Cys Ala Thr Gln Asn Glu
        865             870             875

ATC GAT GAG CAC GAC GCC GAG CTG CTG ATC AAG CAC GGC TGC CAG TAC
1152
Ile Asp Glu His Asp Ala Glu Leu Leu Ile Lys His Gly Cys Gln Tyr
880             885             890             895

GTG GTG GAG GGC GCC AAC ATG CCC TCC ACC AAC GAG GCC ATC CAC AAG
1200
Val Val Glu Gly Ala Asn Met Pro Ser Thr Asn Glu Ala Ile His Lys
                900             905             910

TAC AAC AAG GCC GGC ATC ATC TAC TGC CCC GGC AAG GCG GCC AAC GCC
1248
Tyr Asn Lys Ala Gly Ile Ile Tyr Cys Pro Gly Lys Ala Ala Asn Ala
                915             920             925

GGC GGC GTG GCG GTC AGC GGC CTG GAG ATG ACC CAG AAC CGC ATG AGC
1296
Gly Gly Val Ala Val Ser Gly Leu Glu Met Thr Gln Asn Arg Met Ser
                930             935             940

CTG AAC TGG ACT CGC GAG GAG GTT CGC GAC AAG CTG GAG CGC ATC ATG
1344
Leu Asn Trp Thr Arg Glu Glu Val Arg Asp Lys Leu Glu Arg Ile Met
        945             950             955

AAG GAC ATC TAC GAC TCC GCC ATG GGG CCG TCC CGC AGA TAC AAT GTT
1392
Lys Asp Ile Tyr Asp Ser Ala Met Gly Pro Ser Arg Arg Tyr Asn Val
960             965             970             975

GAC CTG GCT GCG GGC GCC AAC ATC GCG GGC TTC ACC AAG GTG GCT GAT
1440
Asp Leu Ala Ala Gly Ala Asn Ile Ala Gly Phe Thr Lys Val Ala Asp
                980             985             990

GCC GTC AAG GCC CAG GGC GCT GTT TAAGCTGCCC AGGCCCAAGC CACGGCTCAC
1494
Ala Val Lys Ala Gln Gly Ala Val
                995

CGGCAATCCA AC
1506
```

(2) INFORMATION FOR SEQ ID NO:24:

    (i) SEQUENCE CHARACTERISTICS:

        (A) LENGTH: 487 amino acids
        (B) TYPE: amino acid
        (D) TOPOLOGY: linear

    (ii) MOLECULE TYPE: protein
    (xi) SEQUENCE DESCRIPTION: SEQ ID NO:24:

```
Met Ala Val Ser Leu Glu Glu Gln Ile Ser Ala Met Asp Ala Thr Thr
 1               5                   10                      15
```

```
Gly Asp Phe Thr Ala Leu Gln Lys Ala Val Lys Gln Met Ala Thr Lys
            20              25              30

Ala Gly Thr Glu Gly Leu Val His Gly Ile Lys Asn Pro Asp Val Arg
            35              40              45

Gln Leu Leu Thr Glu Ile Phe Met Lys Asp Pro Glu Gln Gln Glu Phe
    50              55              60

Met Gln Ala Val Arg Glu Val Ala Val Ser Leu Gln Pro Val Phe Glu
65              70              75              80

Lys Arg Pro Glu Leu Leu Pro Ile Phe Lys Gln Ile Val Glu Pro Glu
            85              90              95

Arg Val Ile Thr Phe Arg Val Ser Trp Leu Asp Asp Ala Gly Asn Leu
            100             105             110

Gln Val Asn Arg Gly Phe Arg Val Gln Tyr Ser Ser Ala Ile Gly Pro
            115             120             125

Tyr Lys Gly Gly Leu Arg Phe His Pro Ser Val Asn Leu Ser Ile Met
    130             135             140

Lys Phe Leu Ala Phe Glu Gln Ile Phe Lys Asn Ser Leu Thr Thr Leu
145             150             155             160

Pro Met Gly Gly Gly Lys Gly Gly Ser Asp Phe Asp Pro Lys Gly Lys
            165             170             175

Ser Asp Ala Glu Val Met Arg Phe Cys Gln Ser Phe Met Thr Glu Leu
            180             185             190

Gln Arg His Ile Ser Tyr Val Gln Asp Val Pro Ala Gly Asp Ile Gly
            195             200             205

Val Gly Ala Arg Glu Ile Gly Tyr Leu Phe Gly Gln Tyr Lys Arg Ile
    210             215             220

Thr Lys Asn Tyr Thr Gly Val Leu Thr Pro Lys Gly Gln Glu Tyr Gly
225             230             235             240

Gly Ser Glu Ile Arg Pro Glu Ala Thr Gly Tyr Gly Ala Val Leu Phe
            245             250             255

Val Glu Asn Val Leu Lys Asp Lys Gly Glu Ser Leu Lys Gly Lys Arg
            260             265             270

Cys Leu Val Ser Gly Ala Gly Asn Val Ala Gln Tyr Cys Ala Glu Leu
            275             280             285

Leu Leu Glu Lys Gly Ala Ile Val Leu Ser Leu Ser Asp Ser Gln Gly
            290             295             300

Tyr Val Tyr Glu Pro Asn Gly Phe Thr Arg Glu Gln Leu Gln Ala Val
305             310             315             320

Gln Asp Met Lys Lys Lys Asn Asn Ser Ala Arg Ile Ser Glu Tyr Lys
            325             330             335

Ser Asp Thr Ala Val Tyr Val Gly Asp Arg Arg Lys Pro Trp Glu Leu
            340             345             350

Asp Cys Gln Val Asp Ile Ala Phe Pro Cys Ala Thr Gln Asn Glu Ile
            355             360             365
```

```
Asp Glu His Asp Ala Glu Leu Leu Ile Lys His Gly Cys Gln Tyr Val
    370             375             380

Val Glu Gly Ala Asn Met Pro Ser Thr Asn Glu Ala Ile His Lys Tyr
385             390             395                         400

Asn Lys Ala Gly Ile Ile Tyr Cys Pro Gly Lys Ala Ala Asn Ala Gly
            405             410                     415

Gly Val Ala Val Ser Gly Leu Glu Met Thr Gln Asn Arg Met Ser Leu
            420             425             430

Asn Trp Thr Arg Glu Glu Val Arg Asp Lys Leu Glu Arg Ile Met Lys
        435             440             445

Asp Ile Tyr Asp Ser Ala Met Gly Pro Ser Arg Arg Tyr Asn Val Asp
    450             455             460

Leu Ala Ala Gly Ala Asn Ile Ala Gly Phe Thr Lys Val Ala Asp Ala
465             470             475                         480

Val Lys Ala Gln Gly Ala Val
            485
```

(2) INFORMATION FOR SEQ ID NO:25:

   (i) SEQUENCE CHARACTERISTICS:

      (A) LENGTH: 1473 base pairs
      (B) TYPE: nucleic acid
      (C) STRANDEDNESS: double
      (D) TOPOLOGY: linear

   (ii) MOLECULE TYPE: CDNA
   (ix) FEATURE:

      (A) NAME/KEY: CDS
      (B) LOCATION: 4..1431

   (xi) SEQUENCE DESCRIPTION: SEQ ID NO:25:

```
CAT ATG GAC GCC ACC ACC GGC GAC TTC ACG GCG CTG CAG AAG GCG GTG
48
    Met Asp Ala Thr Thr Gly Asp Phe Thr Ala Leu Gln Lys Ala Val
        490             495             500

AAG CAG ATG GCC ACC AAG GCG GGC ACT GAG GGC CTG GTG CAC GGC ATC
96
Lys Gln Met Ala Thr Lys Ala Gly Thr Glu Gly Leu Val His Gly Ile
        505             510             515

AAG AAC CCC GAC GTG CGC CAG CTG CTG ACC GAG ATC TTC ATG AAG GAC
144
Lys Asn Pro Asp Val Arg Gln Leu Leu Thr Glu Ile Phe Met Lys Asp
        520             525             530

CCG GAG CAG CAG GAG TTC ATG CAG GCG GTG CGC GAG GTG GCC GTC TCC
192
Pro Glu Gln Gln Glu Phe Met Gln Ala Val Arg Glu Val Ala Val Ser
535             540             545             550

CTG CAG CCC GTG TTC GAG AAG CGC CCC GAG CTG CTG CCC ATC TTC AAG
240
Leu Gln Pro Val Phe Glu Lys Arg Pro Glu Leu Leu Pro Ile Phe Lys
            555             560             565
```

EP 0 859 849 B1

```
CAG ATC GTT GAG CCT GAG CGC GTG ATC ACC TTC CGC GTG TCC TGG CTG
288
Gln Ile Val Glu Pro Glu Arg Val Ile Thr Phe Arg Val Ser Trp Leu
            570               575               580

GAC GAC GCC GGC AAC CTG CAG GTC AAC CGC GGC TTC CGC GTG CAG TAC
336
Asp Asp Ala Gly Asn Leu Gln Val Asn Arg Gly Phe Arg Val Gln Tyr
        585               590               595

TCG TCC GCC ATC GGC CCC TAC AAG GGC GGC CTG CGC TTC CAC CCC TCC
384
Ser Ser Ala Ile Gly Pro Tyr Lys Gly Gly Leu Arg Phe His Pro Ser
        600               605               610

GTG AAC CTG TCC ATC ATG AAG TTC CTT GCC TTT GAG CAG ATC TTC AAG
432
Val Asn Leu Ser Ile Met Lys Phe Leu Ala Phe Glu Gln Ile Phe Lys
615               620               625               630

AAC AGC CTG ACC ACC CTG CCC ATG GGC GGC GGC AAG GGC GGC TCC GAC
480
Asn Ser Leu Thr Thr Leu Pro Met Gly Gly Gly Lys Gly Gly Ser Asp
            635               640               645

TTC GAC CCC AAG GGC AAG AGC GAC GCG GAG GTG ATG CGC TTC TGC CAG
528
Phe Asp Pro Lys Gly Lys Ser Asp Ala Glu Val Met Arg Phe Cys Gln
            650               655               660

TCC TTC ATG ACC GAG CTG CAG CGC CAC ATC AGC TAC GTG CAG GAC GTG
576
Ser Phe Met Thr Glu Leu Gln Arg His Ile Ser Tyr Val Gln Asp Val
            665               670               675

CCC GCC GGC GAC ATC GGC GTG GGC GCG CGC GAG ATT GGC TAC CTT TTC
624
Pro Ala Gly Asp Ile Gly Val Gly Ala Arg Glu Ile Gly Tyr Leu Phe
            680               685               690

GGC CAG TAC AAG CGC ATC ACC AAG AAC TAC ACC GGC GTG CTG ACC CCG
672
Gly Gln Tyr Lys Arg Ile Thr Lys Asn Tyr Thr Gly Val Leu Thr Pro
695               700               705               710

AAG GGC CAG GAG TAT GGC GGC TCC GAG ATC CGC CCC GAG GCC ACC GGC
720
Lys Gly Gln Glu Tyr Gly Gly Ser Glu Ile Arg Pro Glu Ala Thr Gly
            715               720               725

TAC GGC GCC GTG CTG TTT GTG GAG AAC GTG CTG AAG GAC AAG GGC GAG
768
Tyr Gly Ala Val Leu Phe Val Glu Asn Val Leu Lys Asp Lys Gly Glu
            730               735               740

AGC CTC AAG GGC AAG CGC TGC CTG GTG TCT GGC GCG GGC AAC GTG GCC
816
Ser Leu Lys Gly Lys Arg Cys Leu Val Ser Gly Ala Gly Asn Val Ala
            745               750               755

CAG TAC TGC GCG GAG CTG CTG CTG GAG AAG GGC GCC ATC GTG CTG TCG
864
Gln Tyr Cys Ala Glu Leu Leu Leu Glu Lys Gly Ala Ile Val Leu Ser
        760               765               770
```

48

```
CTG TCC GAC TCC CAG GGC TAC GTG TAC GAG CCC AAC GGC TTC ACG CGC
912
Leu Ser Asp Ser Gln Gly Tyr Val Tyr Glu Pro Asn Gly Phe Thr Arg
775             780             785             790

GAG CAG CTG CAG GCG GTG CAG GAC ATG AAG AAG AAG AAC AAC AGC GCC
960
Glu Gln Leu Gln Ala Val Gln Asp Met Lys Lys Lys Asn Asn Ser Ala
            795             800             805

CGC ATC TCC GAG TAC AAG AGC GAC ACC GCC GTG TAT GTG GGC GAC CGC
1008
Arg Ile Ser Glu Tyr Lys Ser Asp Thr Ala Val Tyr Val Gly Asp Arg
            810             815             820

CGC AAG CCT TGG GAG CTG GAC TGC CAG GTG GAC ATC GCC TTC CCC TGC
1056
Arg Lys Pro Trp Glu Leu Asp Cys Gln Val Asp Ile Ala Phe Pro Cys
            825             830             835

GCC ACC CAG AAC GAG ATC GAT GAG CAC GAC GCC GAG CTG CTG ATC AAG
1104
Ala Thr Gln Asn Glu Ile Asp Glu His Asp Ala Glu Leu Leu Ile Lys
        840             845             850

CAC GGC TGC CAG TAC GTG GTG GAG GGC GCC AAC ATG CCC TCC ACC AAC
1152
His Gly Cys Gln Tyr Val Val Glu Gly Ala Asn Met Pro Ser Thr Asn
855             860             865             870

GAG GCC ATC CAC AAG TAC AAC AAG GCC GGC ATC ATC TAC TGC CCC GGC
1200
Glu Ala Ile His Lys Tyr Asn Lys Ala Gly Ile Ile Tyr Cys Pro Gly
            875             880             885

AAG GCG GCC AAC GCC GGC GGC GTG GCG GTC AGC GGC CTG GAG ATG ACC
1248
Lys Ala Ala Asn Ala Gly Gly Val Ala Val Ser Gly Leu Glu Met Thr
        890             895             900

CAG AAC CGC ATG AGC CTG AAC TGG ACT CGC GAG GAG GTT CGC GAC AAG
1296
Gln Asn Arg Met Ser Leu Asn Trp Thr Arg Glu Glu Val Arg Asp Lys
            905             910             915

CTG GAG CGC ATC ATG AAG GAC ATC TAC GAC TCC GCC ATG GGG CCG TCC
1344
Leu Glu Arg Ile Met Lys Asp Ile Tyr Asp Ser Ala Met Gly Pro Ser
        920             925             930

CGC AGA TAC AAT GTT GAC CTG GCT GCG GGC GCC AAC ATC GCG GGC TTC
1392
Arg Arg Tyr Asn Val Asp Leu Ala Ala Gly Ala Asn Ile Ala Gly Phe
935             940             945             950

ACC AAG GTG GCT GAT GCC GTC AAG GCC CAG GGC GCT GTT TAAGCTGCCC
1441
Thr Lys Val Ala Asp Ala Val Lys Ala Gln Gly Ala Val
            955             960

AGGCCCAAGC CACGGCTCAC CGGCAATCCA AC
1473
```

(2) INFORMATION FOR SEQ ID NO:26:

(i) SEQUENCE CHARACTERISTICS:

(A) LENGTH: 476 amino acids
(B) TYPE: amino acid
(D) TOPOLOGY: linear

(ii) MOLECULE TYPE: protein
(xi) SEQUENCE DESCRIPTION: SEQ ID NO:26:

```
Met Asp Ala Thr Thr Gly Asp Phe Thr Ala Leu Gln Lys Ala Val Lys
 1               5                  10                  15

Gln Met Ala Thr Lys Ala Gly Thr Glu Gly Leu Val His Gly Ile Lys
                20              25                  30

Asn Pro Asp Val Arg Gln Leu Leu Thr Glu Ile Phe Met Lys Asp Pro
        35                  40                  45

Glu Gln Gln Glu Phe Met Gln Ala Val Arg Glu Val Ala Val Ser Leu
    50                  55                  60

Gln Pro Val Phe Glu Lys Arg Pro Glu Leu Leu Pro Ile Phe Lys Gln
65                  70                  75                  80

Ile Val Glu Pro Glu Arg Val Ile Thr Phe Arg Val Ser Trp Leu Asp
                85                  90                  95

Asp Ala Gly Asn Leu Gln Val Asn Arg Gly Phe Arg Val Gln Tyr Ser
            100                 105                 110

Ser Ala Ile Gly Pro Tyr Lys Gly Gly Leu Arg Phe His Pro Ser Val
            115                 120                 125

Asn Leu Ser Ile Met Lys Phe Leu Ala Phe Glu Gln Ile Phe Lys Asn
    130                 135                 140

Ser Leu Thr Thr Leu Pro Met Gly Gly Gly Lys Gly Gly Ser Asp Phe
145                 150                 155                 160

Asp Pro Lys Gly Lys Ser Asp Ala Glu Val Met Arg Phe Cys Gln Ser
            165                 170                 175

Phe Met Thr Glu Leu Gln Arg His Ile Ser Tyr Val Gln Asp Val Pro
            180                 185                 190

Ala Gly Asp Ile Gly Val Gly Ala Arg Glu Ile Gly Tyr Leu Phe Gly
            195                 200                 205

Gln Tyr Lys Arg Ile Thr Lys Asn Tyr Thr Gly Val Leu Thr Pro Lys
    210                 215                 220

Gly Gln Glu Tyr Gly Gly Ser Glu Ile Arg Pro Glu Ala Thr Gly Tyr
225                 230                 235                 240

Gly Ala Val Leu Phe Val Glu Asn Val Leu Lys Asp Lys Gly Glu Ser
            245                 250                 255

Leu Lys Gly Lys Arg Cys Leu Val Ser Gly Ala Gly Asn Val Ala Gln
            260                 265                 270

Tyr Cys Ala Glu Leu Leu Leu Glu Lys Gly Ala Ile Val Leu Ser Leu
    275                 280                 285

Ser Asp Ser Gln Gly Tyr Val Tyr Glu Pro Asn Gly Phe Thr Arg Glu
    290                 295                 300

Gln Leu Gln Ala Val Gln Asp Met Lys Lys Lys Asn Asn Ser Ala Arg
305                 310                 315                 320
```

```
Ile Ser Glu Tyr Lys Ser Asp Thr Ala Val Tyr Val Gly Asp Arg Arg
            325             330             335

Lys Pro Trp Glu Leu Asp Cys Gln Val Asp Ile Ala Phe Pro Cys Ala
            340             345             350

Thr Gln Asn Glu Ile Asp Glu His Asp Ala Glu Leu Leu Ile Lys His
        355             360             365

Gly Cys Gln Tyr Val Val Glu Gly Ala Asn Met Pro Ser Thr Asn Glu
        370             375             380

Ala Ile His Lys Tyr Asn Lys Ala Gly Ile Ile Tyr Cys Pro Gly Lys
385             390             395             400

Ala Ala Asn Ala Gly Gly Val Ala Val Ser Gly Leu Glu Met Thr Gln
            405             410             415

Asn Arg Met Ser Leu Asn Trp Thr Arg Glu Glu Val Arg Asp Lys Leu
            420             425             430

Glu Arg Ile Met Lys Asp Ile Tyr Asp Ser Ala Met Gly Pro Ser Arg
            435             440             445

Arg Ty r Asn Val Asp Leu Ala Ala Gly Ala Asn Ile Ala Gly Phe Thr
        450             455             460

Lys Val Ala Asp Ala Val Lys Ala Gln Gly Ala Val
465             470             475
```